(19) Europäisches Patentamt
European Patent Office
Office européen des brevets

(11) EP 4 506 745 A1

(12) EUROPEAN PATENT APPLICATION

(43) Date of publication:
**12.02.2025 Bulletin 2025/07**

(21) Application number: **24193689.7**

(22) Date of filing: **08.08.2024**

(51) International Patent Classification (IPC):
***G02B 21/18*** (2006.01) ***G02B 21/36*** (2006.01)
***G02B 27/00*** (2006.01) ***G02B 27/10*** (2006.01)

(52) Cooperative Patent Classification (CPC):
**G02B 21/361; G02B 21/18; G02B 27/0075; G02B 27/1013**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.08.2023 CN 202311002370**

(71) Applicant: **GeneMind Biosciences Company Limited**
**Shenzhen City, Guangdong 518023 (CN)**

(72) Inventors:
- **ZHANG, Yongbo**
**Shenzhen City, 518023 (CN)**
- **LI, Yang**
**Shenzhen City, 518023 (CN)**
- **WU, Ping**
**Shenzhen City, 518023 (CN)**
- **JIANG, Zefei**
**Shenzhen City, 518023 (CN)**

(74) Representative: **Schiweck Weinzierl Koch Patentanwälte Partnerschaft mbB**
**Ganghoferstraße 68 B**
**80339 München (DE)**

# (54) IMAGING SYSTEM AND SEQUENCING SYSTEM

(57) The present disclosure discloses an imaging system and a sequencing system. The imaging system comprises an objective lens and image sensors. The image sensor comprises a first set of image sensors and a second set of image sensors. A first set of tube lenses are arranged between the objective lens and the first set of image sensors and are configured for enabling the imaging system to have a first depth of field when imaging a first surface; a second set of tube lenses are arranged between the objective lens and the second set of image sensors and are configured for enabling the imaging system to have a second depth of field when imaging a second surface. The first depth of field and the second depth of field are both smaller than a distance of displacement from the first surface to the second surface along the optical axis of the objective lens. The imaging system of the present disclosure can simultaneously achieve the acquisition of optical signals on two surfaces of a sample of interest and imaging. This allows the imaging system to detect biomolecules on the two surfaces of the sample of interest, identify the type of bases in the biomolecules, and acquire the base sequences, thus reducing the detection time and improving the detection efficiency and the sequencing throughput.

100
70
212(21)
302
407(40)
50
62
62
417(41)
51
222(22)
54
53
312
20
61
52
211(21)
301
62
62
406(40)
111
60
416(41)
311
221(22)
10
200
FIG. 1



Processed by Luminess, 75001 PARIS (FR)

# Description

## TECHNICAL FIELD

The present disclosure relates to the field of gene sequencing, and in particular, to an imaging system and a sequencing system.

## BACKGROUND

In gene sequencing, biomolecules with an optically detectable label immobilized on the surface of a biochip may be excited by excitation light to generate optical signals. The optical signals are detected by an imaging system to give images, and the relevant biological information of the biomolecules may be acquired by analysis of the images. A biochip may have more than one surface on which biomolecules are immobilized. When the surfaces are detected on an optical imaging system, it is difficult to acquire sharp images of the surfaces simultaneously, which reduces the sequencing efficiency and sequencing throughput. Therefore, the simultaneous imaging of multiple surfaces of a biochip is a technical problem to be solved.

## SUMMARY

The present disclosure provides an imaging system and a sequencing system.

The imaging system according to embodiments of the present application is configured for simultaneously imaging a first surface and a second surface of a sample of interest. The imaging system comprises an objective lens and image sensors. The image sensors comprises a first set of image sensors and a second set of image sensors. A first set of tube lenses are arranged between the objective lens and the first set of image sensors and are configured for enabling the imaging system to have a first depth of field when imaging a first surface; a second set of tube lenses are arranged between the objective lens and the second set of image sensors and are configured for enabling the imaging system to have a second depth of field when imaging a second surface. The first depth of field and the second depth of field are both smaller than a distance of displacement from the first surface to the second surface along the optical axis of the objective lens.

The imaging system of the present disclosure can simultaneously achieve the acquisition of optical signals on two surfaces of a sample of interest and imaging. This allows the imaging system to detect biomolecules on the two surfaces of the sample of interest, identify the types of bases in the biomolecules, and acquire the base sequences of the biomolecules, thus reducing the detection time and improving the detection efficiency and the sequencing throughput. In addition, the first set of image sensors and the second set of image sensors can image on the basis of the optical signals on the first surface of the sample of interest and the optical signals on the second surface of the sample of interest, respectively, thus reducing the subsequent processing algorithms of the images and further improving the sequencing efficiency. The first set of tube lenses and the second set of tube lenses can simultaneously and respectively converge the optical signals on the first surface and the second surface of the sample of interest, such that the first set of image sensors and the second set of image sensors can simultaneously and respectively acquire the optical signals on the first surface and the second surface of the sample of interest and image. The first depth of field and the second depth of field enable the two surfaces of the sample of interest to have their corresponding depth of field ranges, and the imaging processes of the two surfaces do not interfere with each other and thus require no extra image algorithm processing for calling base.

In some embodiments, the first depth of field ranges from 1.5 μm to 1.8 μm, and the second depth of field ranges from 1.5 μm to 1.8 μm.

The aforementioned depth of field ranges allow sharp imaging by the first set of image sensors and the second set of image sensors on the basis of the optical signals on two surfaces of the sample of interest.

In some embodiments, the distance of displacement from the first surface to the second surface along the optical axis of the objective lens ranges from 40 μm to 60 μm.

When the first surface and the second surface are within the above distance ranges, the first depth of field and the second depth of field are both less than the perpendicular distance between the first surface and the second surface, preventing the optical signals on the first surface and the optical signals on the second surface from interfering with each other when imaging.

In some embodiments, the objective lens has a numerical aperture greater than 0.6 and a field of view greater than 1.2 mm.

The aperture and the field of view are beneficial to detecting a greater area on the sample of interest, thus allowing the system to provide more comprehensive and detailed measurement results and more information. As such, the imaging system may fit a greater range of optical devices.

In some embodiments, the combination of the objective lens and the first set of tube lenses has a distortion of less than 0.5.

As such, the image generated by the first set of image sensors is less distorted, thereby ensuring the accuracy of the sequencing biomolecules on the first surface.

In some embodiments, the combination of the objective lens and the second set of tube lenses has a distortion of less than 0.2.

As such, the image generated by the second set of image sensors is less distorted, thereby ensuring the accuracy of the sequencing biomolecules on the second surface.

In some embodiments, the first set of tube lenses comprise a first optical lens, a second optical lens, a third optical lens, a fourth optical lens, and a fifth optical lens in sequence, wherein the first optical lens has a negative refractive power, the second optical lens has a positive refractive power, the third optical lens has a positive refractive power, the fourth optical lens has a positive refractive power, and the fifth optical lens has a negative refractive power; the object-side surfaces and the image-side surfaces of the first optical lens, the second optical lens, the third optical lens, the fourth optical lens, and the fifth optical lens are all spherical surfaces, and the object-side surface of one optical lens and the image-side surface of the other optical lens in each pair of adjacent optical lenses are arranged towards each other.

According to embodiments of the present application, the first set of tube lenses comprise five optical lenses and thus feature compactness; the object-side surfaces and the image-side surfaces of the five optical lenses are all spherical surfaces, allowing the first set of tube lenses to greatly reduce the aberration in a visible wavelength range of 553 nm to 712 nm. Compared with aspherical optical lenses, the optical lenses according to embodiments of the present application feature ease to manufacture, and thereby provide the first set of tube lenses ease to manufacture. The combination of the first set of tube lenses with an infinite conjugate objective lens can sharply converge optical signals from the first surface on an image surface of the first set of image sensors.

In some embodiments, the object-side surface of the first optical lens is convex at the optical axis of the first optical lens, and the image-side surface of the first optical lens is concave at the optical axis of the first optical lens; the object-side surface of the first optical lens has a curvature radius of 160 mm to 190 mm at the optical axis of the first optical lens, and the image-side surface of the first optical lens has a curvature radius of 60 mm to 90 mm at the optical axis of the first optical lens.

When the first optical lens meets the curvature radius, the design difficulty and the assembly sensitivity of the first set of tube lenses can be reduced.

In some embodiments, the object-side surface of the second optical lens is convex at the optical axis of the second optical lens, and the image-side surface of the second optical lens is convex at the optical axis of the second optical lens; the object-side surface of the second optical lens has a curvature radius of 60 mm to 90 mm at the optical axis of the second optical lens, and the image-side surface of the second optical lens has a curvature radius of -170 mm to -140 mm at the optical axis of the second optical lens.

When the second optical lens meets the curvature radius, the design difficulty and the assembly sensitivity of the first set of tube lenses can be reduced.

In some embodiments, the object-side surface of the third optical lens is convex at the optical axis of the third optical lens, and the image-side surface of the third optical lens is concave at the optical axis of the third optical lens; the object-side surface of the third optical lens has a curvature radius of 40 mm to 70 mm at the optical axis of the third optical lens, and the image-side surface of the third optical lens has a curvature radius of 110 mm to 140 mm at the optical axis of the third optical lens.

When the third optical lens meets the curvature radius, the design difficulty and the assembly sensitivity of the first set of tube lenses can be reduced.

In some embodiments, the object-side surface of the fourth optical lens is convex at the optical axis of the fourth optical lens, and the image-side surface of the fourth optical lens is convex at the optical axis of the fourth optical lens; the object-side surface of the fourth optical lens has a curvature radius of 30 mm to 50 mm at the optical axis of the fourth optical lens, and the image-side surface of the fourth optical lens has a curvature radius of -700 mm to -650 mm at the optical axis of the fourth optical lens.

When the fourth optical lens meets the curvature radius, the design difficulty and the assembly sensitivity of the first set of tube lenses can be reduced.

In some embodiments, the object-side surface of the fifth optical lens is concave at the optical axis of the fifth optical lens, and the image-side surface of the fifth optical lens is concave at the optical axis of the fifth optical lens; the object-side surface of the fifth optical lens has a curvature radius of -700 mm to -650 mm at the optical axis of the fifth optical lens, and the image-side surface of the fifth optical lens has a curvature radius of 20 mm to 40 mm at the optical axis of the fifth optical lens.

When the fifth optical lens meets the curvature radius, the design difficulty and the assembly sensitivity of the first set of tube lenses can be reduced.

In some embodiments, the first optical lens has a thickness of 2 mm to 5 mm at the optical axis of the first optical lens, the second optical lens has a thickness of 9 mm to 13 mm at the optical axis of the second optical lens, the third optical lens has a thickness of 6 mm to 10 mm at the optical axis of the third optical lens, the fourth optical lens has a thickness of 9 mm to 14 mm at the optical axis of the fourth optical lens, and the fifth optical lens has a thickness of 3 mm to 7 mm at the optical axis of the fifth optical lens.

Through the proper configuration of the thicknesses of different optical lenses, the optimal balance between the miniaturization of the first set of tube lenses and the manufacturability of the optical lenses may be achieved, thereby avoiding compromised strength of the first set of tube lenses due to excessively thin optical lenses and thus affected fabrication yield.

In some embodiments, the first optical lens and the second optical lens are cemented to form an optical lens set, and the fourth optical lens and the fifth optical lens are cemented to form an optical lens set.

Through the cemented optical lenses, the optical axes of the first optical lens and the second optical lens are aligned, and the optical axes of the fourth optical lens and the fifth optical lens are aligned, thus eliminating the chromatic aberration between the first optical lens and the second optical lens and the chromatic aberration between the fourth optical lens and the fifth optical lens and facilitating the assembly and the calibration of the first set of tube lenses.

In some embodiments, the second set of tube lenses comprise a sixth optical lens, a seventh optical lens, an eighth optical lens, a ninth optical lens, and a tenth optical lens in sequence, wherein the sixth optical lens has a negative refractive power, the seventh optical lens has a positive refractive power, the eighth optical lens has a positive refractive power, the ninth optical lens has a positive refractive power, and the tenth optical lens has a negative refractive power; the object-side surfaces and the image-side surfaces of the sixth optical lens, the seventh optical lens, the eighth optical lens, the ninth optical lens, and the tenth optical lens are all spherical surfaces, and the object-side surface of one lens and the image-side surface of the other lens in each pair of adjacent lenses are arranged towards each other.

According to embodiments of the present application, the second set of tube lenses comprise five lenses and thus feature compactness; the object-side surfaces and the image-side surfaces of the five lenses are all spherical surfaces, allowing the second set of tube lenses to greatly reduce the aberration in a visible wavelength range of 553 nm to 712 nm. Compared with aspherical lenses, the lenses according to embodiments of the present application feature ease to manufacture, and thereby provide the second set of tube lenses ease to manufacture. The combination of the second set of tube lenses with an infinite conjugate objective lens can sharply converge optical signals from the second surface on an image surface of the second set of image sensors.

In some embodiments, the object-side surface of the sixth optical lens is convex at the optical axis of the sixth optical lens, and the image-side surface of the sixth optical lens is concave at the optical axis of the sixth optical lens; the object-side surface of the sixth optical lens has a curvature radius of 110 mm to 130 mm at the optical axis of the sixth optical lens, and the image-side surface of the sixth optical lens has a curvature radius of 40 mm to 70 mm at the optical axis of the sixth optical lens.

When the sixth optical lens meets the curvature radius, the design difficulty and the assembly sensitivity of the second set of tube lenses can be reduced.

In some embodiments, the object-side surface of the seventh optical lens is convex at the optical axis of the seventh optical lens, and the image-side surface of the seventh optical lens is convex at the optical axis of the seventh optical lens; the object-side surface of the seventh optical lens has a curvature radius of 40 mm to 70 mm at the optical axis of the seventh optical lens, and the image-side surface of the seventh optical lens has a curvature radius of -350 mm to -300 mm at the optical axis of the seventh optical lens.

When the seventh optical lens meets the curvature radius, the design difficulty and the assembly sensitivity of the second set of tube lenses can be reduced.

In some embodiments, the object-side surface of the eighth optical lens is convex at the optical axis of the eighth optical lens, and the image-side surface of the eighth optical lens is concave at the optical axis of the eighth optical lens; the object-side surface of the eighth optical lens has a curvature radius of 50 mm to 70 mm at the optical axis of the eighth optical lens, and the image-side surface of the eighth optical lens has a curvature radius of 180 mm to 220 mm at the optical axis of the eighth optical lens.

When the eighth optical lens meets the curvature radius, the design difficulty and the assembly sensitivity of the second set of tube lenses can be reduced.

In some embodiments, the object-side surface of the ninth optical lens is convex at the optical axis of the ninth optical lens, and the image-side surface of the ninth optical lens is convex at the optical axis of the ninth optical lens; the object-side surface of the ninth optical lens has a curvature radius of 40 mm to 60 mm at the optical axis of the ninth optical lens, and the image-side surface of the ninth optical lens has a curvature radius of -150 mm to -110 mm at the optical axis of the ninth optical lens.

When the ninth optical lens meets the curvature radius, the design difficulty and the assembly sensitivity of the second set of tube lenses can be reduced.

In some embodiments, the object-side surface of the tenth optical lens is concave at the optical axis of the tenth optical lens, and the image-side surface of the tenth optical lens is concave at the optical axis of the tenth optical lens; the object-side surface of the tenth optical lens has a curvature radius of -150 mm to -110 mm at the optical axis of the tenth optical lens, and the image-side surface of the tenth optical lens has a curvature radius of 20 mm to 50 mm at the optical axis of the tenth optical lens.

When the tenth optical lens meets the curvature radius, the design difficulty and the assembly sensitivity of the second set of tube lenses can be reduced.

In some embodiments, the sixth optical lens has a thickness of 3 mm to 7 mm at the optical axis of the sixth optical lens, the seventh optical lens has a thickness of 9 mm to 13 mm at the optical axis of the seventh optical lens, the eighth optical lens has a thickness of 7 mm to 10 mm at the optical axis of the eighth optical lens, the ninth optical lens has a thickness of 10 mm to 15 mm at the optical axis of the ninth optical lens, and the tenth optical lens has a thickness of 4 mm to 7 mm at the optical axis of the tenth optical lens.

Through the proper configuration of the thicknesses of different lenses, the optimal balance between the miniaturization of the second set of tube lenses and the manufacturability of the lenses may be achieved, thereby avoiding compromised strength of the second set of tube lenses due to excessively thin lenses and thus affected fabrication yield.

In some embodiments, the sixth optical lens and the seventh optical lens are cemented to form an optical lens set, and the ninth optical lens and the tenth optical lens are cemented to form an optical lens set.

Through the cemented lenses, the optical axes of the sixth optical lens and the seventh optical lens are aligned, and the optical axes of the ninth optical lens and the tenth optical lens are aligned, thus eliminating the chromatic aberration between the sixth optical lens and the seventh optical lens and the chromatic aberration between the ninth optical lens and the tenth optical lens and facilitating the assembly and the calibration of the second set of tube lenses.

In some embodiments, a beamsplitter assembly is arranged between the objective lens and the image sensors; the beamsplitter assembly is configured for splitting optical signals acquired by the objective lens, so as to simultaneously deliver a first optical signal generated by the first surface to the first set of image sensors and a second optical signal generated by the second surface to the second set of image sensors.

As such, the beamsplitter assembly splits the light beam from the objective lens into a plurality of light beams and converges the light beams to corresponding image sensors for imaging, so as to achieve simultaneous detection of different light beams emitted from the two surfaces of the sample of interest.

In some embodiments, the first set of image sensors at least comprise a first image sensor and a second image sensor; the first set of tube lenses at least comprise a first tube lens and a second tube lens that are identical; the beamsplitter assembly is configured for splitting the first optical signal into a light beam with a first wavelength and a light beam with a second wavelength, and simultaneously delivering the light beam with the first wavelength to the first image sensor through the first tube lens and the light beam with the second wavelength to the second image sensor through the second tube lens.

As such, the first image sensor and the second image sensor can separately image, so as to detect the first optical signal emitted by the first surface of the sample of interest. The two image sensors can simultaneously image on the basis of the optical signals with two wavelengths, thus improving the imaging efficiency for various optical signals.

In some embodiments, the second set of image sensors at least comprise a third image sensor and a fourth image sensor; the second set of tube lenses at least comprise a third tube lens and a fourth tube lens that are identical; the beamsplitter assembly is configured for splitting the second optical signal into a light beam with a third wavelength and a light beam with a fourth wavelength, and simultaneously delivering the light beam with the third wavelength to the third image sensor through the third tube lens and the light beam with the fourth wavelength to the fourth image sensor through the fourth tube lens.

As such, the third image sensor and the fourth image sensor can separately image, so as to detect the second optical signal emitted by the second surface of the sample of interest. The two image sensors can simultaneously image on the basis of the optical signals with two wavelengths, thus improving the imaging efficiency for various optical signals.

In some embodiments, the beamsplitter assembly comprises a first dichroic mirror, a first beamsplitter, and a second beamsplitter; the first dichroic mirror is configured for reflecting the light beam with the first wavelength to the first beamsplitter and transmitting the light beam with the second wavelength to the second beamsplitter; the first beamsplitter is configured for receiving the light beam with the first wavelength reflected from the first dichroic mirror and reflecting the light beam with the first wavelength to the first tube lens, so as to deliver the light beam with the first wavelength to the first image sensor through the first tube lens; the second beamsplitter is configured for receiving the light beam with the second wavelength transmitted from the first dichroic mirror and reflecting the light beam with the second wavelength to the second tube lens, so as to deliver the light beam with the second wavelength to the second image sensor through the second tube lens.

As such, the beamsplitter assembly can separate optical signals with different wave bands and different throughputs, allowing the first image sensor to image on the basis of the light beam with the first wavelength and the second image sensor to image on the basis of the light beam with the second wavelength simultaneously.

In some embodiments, the first dichroic mirror is configured for reflecting the light beam with the third wavelength to the first beamsplitter and transmitting the light beam with the fourth wavelength to the second beamsplitter; the first beamsplitter is configured for receiving the light beam with the third wavelength reflected from the first dichroic mirror and transmitting the light beam with the third wavelength to the third tube lens, so as to deliver the light beam with the third wavelength to the third image sensor through the third tube lens; the second beamsplitter is configured for receiving the light beam with the fourth wavelength transmitted from the first dichroic mirror and transmitting the light beam with the fourth wavelength to the fourth tube lens, so as to deliver the light beam with the fourth wavelength to the fourth image sensor through the fourth tube lens.

As such, the beamsplitter assembly can separate optical signals with different wave bands and different throughputs, allowing the third image sensor to image on the basis of the light beam with the third wavelength and the fourth image sensor to image on the basis of the light beam with the fourth wavelength simultaneously.

In some embodiments, the imaging system comprises a light source and a second dichroic mirror arranged between the light source and the objective lens, wherein the second dichroic mirror is arranged on the optical axis of the objective lens, the light source is configured for emitting an excitation light beam, and the second dichroic mirror is configured for reflecting the excitation light beam toward the objective lens, such that the excitation light beam is projected through the objective lens to the first surface and the second surface.

As such, the light source can excite the bases with an optically detectable label binding to the biomolecule to generate an optical signal, thus achieving the base sequence determination of the biomolecule.

In some embodiments, the imaging system comprises an autofocus apparatus, wherein the autofocus apparatus is configured for detecting the positional relationship between the sample of interest and the objective lens and for positioning the sample of interest in the focal plane of the objective lens according to the detected relative positions of the sample of interest and the objective lens.

As such, the imaging system can always guarantee sharp focusing and ensure the accuracy of acquired images.

The sequencing system according to embodiments of the present application comprises the imaging system. The imaging system of the present disclosure can simultaneously achieve the acquisition of optical signals on two surfaces of a sample of interest and imaging. This allows the imaging system to simultaneously sequence biomolecules on the two surfaces of the sample of interest during the gene sequencing process, thus reducing the sequencing time and improving the sequencing efficiency.

Additional aspects and advantages of the present disclosure will in part be illustrated in the following description and become apparent from the following description, or may be learned by practice of the present disclosure.

## BRIEF DESCRIPTION OF THE DRAWINGS

The aforementioned and/or additional aspects and advantages of the present disclosure will become apparent and easily understood from the description of the embodiments with reference to the following drawings, in which:

FIG. 1 illustrates a structural schematic of the imaging system according to embodiments of the present disclosure;

FIG. 2 illustrates a structural schematic of a sample according to embodiments of the present disclosure;

FIG. 3 illustrates a structural schematic of a sample according to embodiments of the present disclosure;

FIG. 4 illustrates a structural schematic of a sample according to embodiments of the present disclosure;

FIG. 5 illustrates a focusing diagram of the imaging system according to embodiments of the present disclosure;

FIG. 6 illustrates distortion curves of the combination of the first set of tube lenses with the objective lens according to embodiments of the present disclosure;

FIG. 7 illustrates distortion curves of the combination of the second set of tube lenses with the objective lens according to embodiments of the present disclosure;

FIG. 8 illustrates a structural schematic of the first set of tube lenses according to embodiments of the present disclosure;

FIG. 9 illustrates MTF curves of the combination of the first set of tube lenses with the objective lens according to embodiments of the present disclosure;

FIG. 10 illustrates MTF curves of the combination of the first set of tube lenses with the objective lens according to embodiments of the present disclosure;

FIG. 11 illustrates MTF curves of the combination of the first set of tube lenses with the objective lens according to embodiments of the present disclosure;

FIG. 12 illustrates MTF curves of the combination of the first set of tube lenses with the objective lens according to embodiments of the present disclosure;

FIG. 13 illustrates MTF curves of the combination of the first set of tube lenses with the objective lens according to embodiments of the present disclosure;

FIG. 14 illustrates wavefront aberration curves of the combination of the first set of tube lenses with the objective lens according to embodiments of the present disclosure;

FIG. 15 illustrates a spot diagram of the combination of the first set of tube lenses with the objective lens according to embodiments of the present disclosure;

FIG. 16 illustrates a focal shift curve of the combination of the first set of tube lenses with the objective lens according to embodiments of the present disclosure;

FIG. 17 illustrates a structural schematic of the second set of tube lenses according to embodiments of the present disclosure;

FIG. 18 illustrates MTF curves of the combination of the second set of tube lenses with the objective lens according to embodiments of the present disclosure;

FIG. 19 illustrates MTF curves of the combination of the second set of tube lenses with the objective lens according to embodiments of the present disclosure;

FIG. 20 illustrates MTF curves of the combination of the second set of tube lenses with the objective lens according to embodiments of the present disclosure;

FIG. 21 illustrates MTF curves of the combination of the second set of tube lenses with the objective lens according to embodiments of the present disclosure;

FIG. 22 illustrates MTF curves of the combination of the second set of tube lenses with the objective lens according to embodiments of the present disclosure;

FIG. 23 illustrates MTF curves of the combination of the second set of tube lenses with the objective lens according to embodiments of the present disclosure;

FIG. 24 illustrates MTF curves of the combination of the second set of tube lenses with the objective lens according to embodiments of the present disclosure;

FIG. 25 illustrates MTF curves of the combination of the second set of tube lenses with the objective lens according to embodiments of the present disclosure;

FIG. 26 illustrates MTF curves of the combination of the second set of tube lenses with the objective lens according to embodiments of the present disclosure;

FIG. 27 illustrates MTF curves of the combination of the second set of tube lenses with the objective lens according to embodiments of the present disclosure;

FIG. 28 illustrates MTF curves of the combination of the second set of tube lenses with the objective lens according to embodiments of the present disclosure;

FIG. 29 illustrates MTF curves of the combination of the second set of tube lenses with the objective lens according to embodiments of the present disclosure;

FIG. 30 illustrates MTF curves of the combination of the second set of tube lenses with the objective lens according to embodiments of the present disclosure;

FIG. 31 illustrates wavefront aberration curves of the combination of the second set of tube lenses with the objective lens according to embodiments of the present disclosure;

FIG. 32 illustrates a spot diagram of the combination of the second set of tube lenses with the objective lens according to embodiments of the present disclosure; and

FIG. 33 illustrates a focal shift curve of the combination of the second set of tube lenses with the objective lens according to embodiments of the present disclosure.

Description of reference numerals: 100, imaging system; 10, objective lens; 20, image sensor; 21, first set of image sensors; 211, first image sensor; 212, second image sensor; 22, second set of image sensors; 221, third image sensor; 222, fourth image sensor; 30, first optical signal; 301, light beam with first wavelength; 302, light beam with second wavelength; 31, second optical signal; 311, light beam with third wavelength; 312, light beam with fourth wavelength; 40, first set of tube lenses; 401, first optical lens; 4011, object-side surface of first optical lens; 4012, image-side surface of first optical lens; 402, second optical lens; 4021, object-side surface of second optical lens; 4022, image-side surface of second optical lens; 403, third optical lens; 4031, object-side surface of third optical lens; 4032, image-side surface of third optical lens; 404, fourth optical lens; 4041, object-side surface of fourth optical lens; 4042, image-side surface of fourth optical lens; 405, fifth optical lens; 4051, object-side surface of fifth optical lens; 4052, image-side surface of fifth optical lens; 406, first tube lens; 407, second tube lens; 41, second set of tube lenses; 411, sixth optical lens; 4111, object-side surface of sixth optical lens; 4112, image-side surface of sixth optical lens; 412, seventh optical lens; 4121, object-side surface of seventh optical lens; 4122, image-side surface of seventh optical lens; 413, eighth optical lens; 4131, object-side surface of eighth optical lens; 4132, image-side surface of eighth optical lens; 414, ninth optical lens; 4141, object-side surface of ninth optical lens; 4142, image-side surface of ninth optical lens; 415, tenth optical lens; 4151, object-side surface of tenth optical lens; 4152, image-side surface of tenth optical lens; 416, third tube lens; 417, fourth tube lens; 50, beamsplitter assembly; 51, first dichroic mirror; 52, first beamsplitter; 53, second beamsplitter; 54, mirror; 60, light source; 61, second dichroic mirror; 62, filter; 70, autofocus apparatus; 200, sample; 201, first substrate; 202, first liquid layer; 203, second substrate; 204, second liquid layer; 210, first surface; 220, second surface.

## DETAILED DESCRIPTION

The embodiments of the present disclosure are described in detail below, and the examples of the embodiments are shown in the accompanying drawings, throughout which identical or similar reference numerals represent identical or similar elements or elements having identical or similar functionality. The embodiments described below by reference to the accompanying drawings are exemplary and illustrative, and should not be construed as limiting the present disclosure.

In the description of the present disclosure, it will be appreciated that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", and the like are those shown on the basis of the accompanying drawings, and are merely intended to facilitate and simplify the description rather than indicate or imply that the indicated device or element must have a specific orientation and be configured and operated according to the specific orientation. Such relationships should not be construed as limiting the present disclosure. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present disclosure, unless otherwise specifically defined, "a plurality of" means two or more.

In the description of the present application, it should be noted that unless otherwise clearly specified and defined, the terms "mount", "link", and "connect" should be interpreted in their broad sense. For example, the connection may be a fixed connection, detachable connection, or integral connection; a mechanic connection, electric connection, or communicative connection; or a direct connection, indirect connection through an intermediate, internal communication of two elements, or interaction between two elements. For those of ordinary skills in the art, the specific meanings of the aforementioned terms in the present disclosure can be interpreted according to specific conditions.

In the present disclosure, unless otherwise clearly specified and defined, a first feature being "above" or "below" a second feature may encompass that the first and second features are in direct contact, and that the first and second features are not in direct contact, but are in contact via an additional feature between them. Also, a first feature being "on", "over" and "above" a second feature encompasses that the first feature is right above or obliquely above the second feature, or simply means that the first feature is at a vertically higher position than the second feature. A first feature being "under", "beneath" and "below" a second feature encompasses that the first feature is right below or obliquely below the second feature, or simply means that the first feature is at a vertically lower position than the second feature.

The following disclosure provides many different embodiments or examples for implementing different structures of the present disclosure. To simplify the disclosure of the present application, the components and settings of specific examples are described below. Certainly, the examples are merely exemplary and are not intended to limit the present disclosure. In addition, reference numerals and/or characters may be repeatedly used in different examples in the present disclosure for simplicity and clarity rather than for indicating the relationship between various embodiments and/or settings discussed. In addition, the present disclosure provides examples of various specific processes and materials, but those of ordinary skills in the art will recognize the application of other processes and/or the use of other materials.

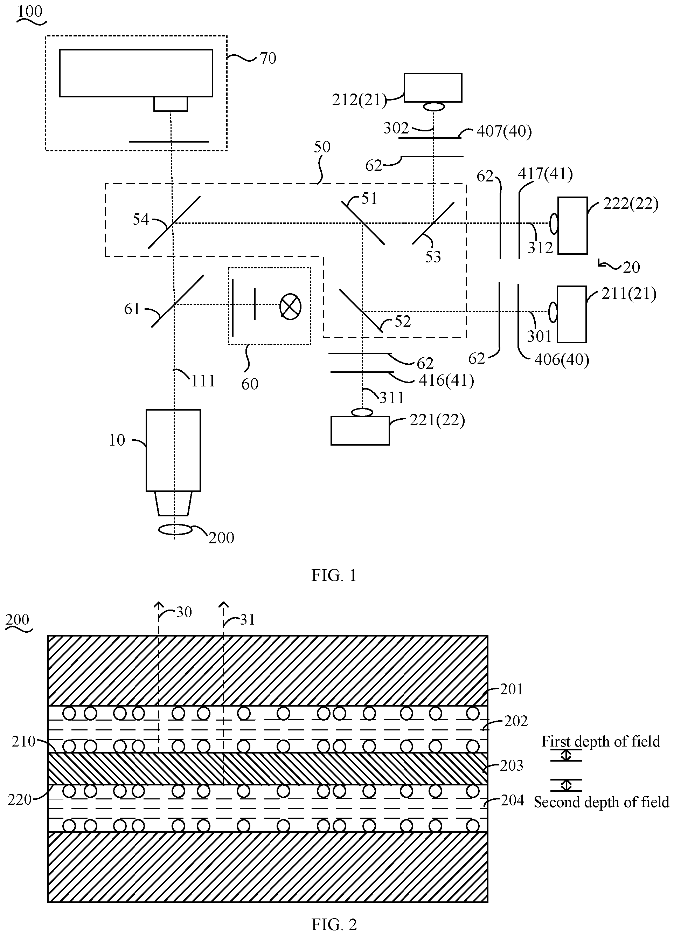

Referring to FIGs. 1 and 2, an imaging system 100 according to embodiments of the present application is used to simultaneously image biomolecules on a first surface 210 and a second surface 220 of a sample 200 of interest. Bases (e.g., A, T, C, and G) with an optically detectable label are bonded to the biomolecules (e.g., nucleic acid molecules), and the bases with an optically detectable label are bonded to bases on the biomolecules according to the complementary base pairing principle. Under the excitation of excitation light, the optically detectable labels on the bases generate an optical signal, and the optical signal is acquired by the imaging system 100, which may identify the types of bases of the biomolecule and determine the base sequence of the biomolecule.

In some embodiments, the optically detectable label is a fluorophore, for example, Cy3, Cy5, Cy7. The fluorophore generates fluorescence under the excitation of excitation light.

Referring to FIGs. 1 and 2, the imaging system 100 comprises an objective lens 10 and image sensors 20, and the image sensors 20 comprise a first set of image sensors 21 and a second set of image sensors 22. A first set of tube lenses 40 are arranged between the objective lens 10 and the first set of image sensors 21 and are configured for enabling the imaging system 100 to have a first depth of field when imaging the first surface 210; a second set of tube lenses 41 are arranged between the objective lens 10 and the second set of image sensors 22 and are configured for enabling the imaging system 100 to have a second depth of field when imaging the second surface 220. The first depth of field and the second depth of field are both smaller than a distance of displacement from the first surface 210 to the second surface 220 along the optical axis of the objective lens 10.

The imaging system 100 of the present disclosure can simultaneously achieve the acquisition of optical signals on two surfaces of the sample 200 of interest and imaging. This allows the imaging system 100 to detect biomolecules on the two surfaces of the sample 200 of interest, identify the types of bases in the biomolecules, and acquire the base sequences of the biomolecules, thus reducing the detection time and improving the detection efficiency and the sequencing throughput. In addition, the first set of image sensors 21 and the second set of image sensors 22 can image on the basis of a first optical signal 30 and a second optical signal 31, respectively, thus reducing the subsequent processing algorithms of the images and further improving the sequencing efficiency.

Compared with a single-surface imaging process, the imaging system 100 can double the throughput in the same period via simultaneous double-surface imaging and halve the photographing time with the throughput being unchanged.

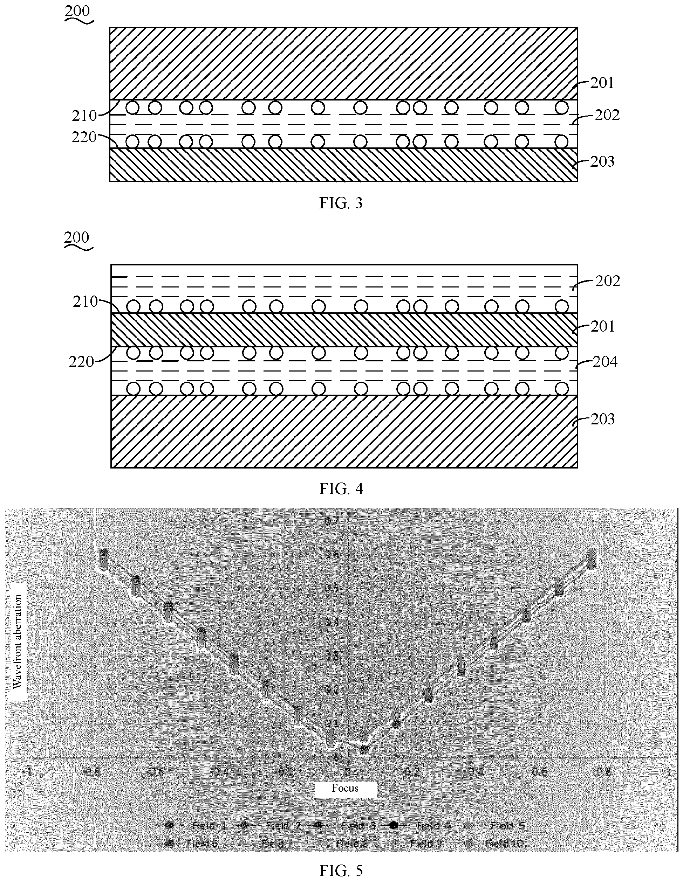

Specifically, referring to FIGs. 2 to 4, taking a sequencing chip sample 200 as an example, the chip may comprise substrates arranged in a stacked manner, and liquid layers may be arranged between the substrates. The biomolecules are located at an interface between the liquid layer and the substrate. Referring to FIG. 2, the first surface 210 is the interface between a second substrate 203 and a first liquid layer 202, and the second surface 220 is the interface between the second substrate 203 and a second liquid layer 204. Referring to FIG. 3, the first surface 210 is the interface between a first substrate 201 and the first liquid layer 202, and the second surface 220 is the interface between the second substrate 203 and the first liquid layer 202. Referring to FIG. 4, the first surface 210 is the interface between the first liquid layer 202 and the first substrate 201, and the second surface 220 is the interface between the first substrate 201 and the second liquid layer 204.

In some embodiments, the objective lens 10 is configured for acquiring the first optical signal 30 from the first surface 210 and the second optical signal 31 from the second surface 220. The objective lens 10 may be an available objective lens, such as an infinite conjugate objective lens, or the objective lens 10 may be a customized objective lens. The focal length of the objective lens 10 can be set according to the requirements of the imaging system 100. The optical axis of the objective lens 10 is perpendicular to the first surface 210 and the second surface 220.

The image sensors 20 are any sensors capable of sensing optical image information and converting same into usable output signals, including but not limited to CCD and CMOS. The first set of image sensors 21 are devices configured for acquiring the first optical signal 30 acquired by the objective lens 10 and generating an image, and may comprise one or more sensors; the second set of image sensors 22 are devices configured for acquiring the second optical signal 31 acquired by the objective lens 10 and generating an image, and may comprise one or more sensors. The first set of image sensors 21 and the second set of image sensors 22 can image on the basis of the optical signals from the first surface 210 and the second surface 220 respectively, and the imaging processes of the two surfaces do not interfere with each other, thus requiring no processings such as extraction, separation, and the like to give two images.

The first set of tube lenses 40 and the second set of tube lenses 41 can simultaneously and respectively converge the optical signals on the first surface 210 and the second surface 220 of the sample 200 of interest, such that the first set of image sensors 21 and the second set of image sensors 22 can simultaneously and respectively acquire the optical signals on the first surface 210 and the second surface 220 of the sample 200 of interest and image. The first set of tube lenses 40 are arranged at the object side of the first set of image sensors 21, and cooperate with the objective lens 10 to converge the first optical signal 30 onto the first set of image sensors 21; the first set of tube lenses 40 may be designed to have different focal lengths according to the imaging requirements. The second set of tube lenses 41 are arranged on the object side of the second set of image sensors 22, and cooperate with the objective lens 10 to converge the second optical signal 31 onto the second set of image sensors 22; the second set of tube lenses 41 may be designed to have different focal lengths according to the imaging requirements. By designing two different tube lenses fitting the objective lens 10, two surfaces of the sample 200 of interest can be imaged simultaneously.

The first depth of field and the second depth of field enable the two surfaces of the sample 200 of interest to have their corresponding depth of field ranges, and the imaging processes of the two surfaces do not interfere with each other and thus require no extra image algorithm processing for base calling. Referring to FIG. 2, the depth of field refers to the range of fluctuation around the focal plane when the imaging system 100 can acquire a sharp image. The depth of field is calculated by the formula: $\mathrm{DOF} = \frac{\lambda * n}{NA^2} + \frac{n}{M * NA} * 2e$, wherein, DOF is the depth of field, $\lambda$ is the wavelength of incident light, NA is the numerical aperture of the objective lens 10, M is the magnification factor, n is the refractive index of air, and e is the size of the camera pixel. In some embodiments, the first depth of field may be as large as the second depth of field, the first depth of field may be larger than the second depth of field, or the first depth of field may also be smaller than the second depth of field.

In some embodiments, the first depth of field ranges from 1.5 to 1.8, and the second depth of field ranges from 1.5 μm to 1.8 μm.

The aforementioned depth of field ranges allow sharp imaging by the first set of image sensors 21 and the second set of image sensors 22 on the basis of the optical signals on two surfaces of the sample 200.

Specifically, when the range of $\lambda$ is 0.553 μm to 0.712 μm, NA is 0.8, M is 13, n is 1.0, and e is 3.45 μm, the calculated DOF range is 1.528 μm to 1.776 μm according to the calculation formula for the depth of field. The first depth of field may be 1.55 μm, 1.60 μm, 1.65 μm, 1.70 μm, 1.75 μm, etc., and the second depth of field may be 1.52 μm, 1.57 μm, 1.62 μm, 1.67 μm, 1.72 μm, or 1.77 μm.

In some embodiments, the distance of displacement from the first surface 210 to the second surface 220 along the optical axis of the objective lens 10 ranges from 40 μm to 60 μm, i.e., the perpendicular distance between the first surface 210 and the second surface 220 is 40 μm to 60 μm.

When the first surface 210 and the second surface 220 are within the above distance ranges, the first depth of field and the second depth of field are both less than the perpendicular distance between the first surface 210 and the second surface 220, preventing the first optical signals on the first surface 210 and the second optical signals on the second surface 220 from interfering with each other when imaging.

Specifically, when the perpendicular distance between the first surface 210 and the second surface 220 is less than 40 μm, the spherical aberration of the image generated by the image sensors 20 may be too large and affect the imaging effects; when the perpendicular distance between the first surface 210 and the second surface 220 is greater than 60 μm, the imaging system 100 may have difficulties in focusing.

Referring to FIG. 1, in some embodiments, a beamsplitter assembly 50 is arranged between the objective lens 10 and the image sensors 20; the beamsplitter assembly 50 is configured for splitting optical signals acquired by the objective lens 10, so as to simultaneously deliver the first optical signal 30 generated by the first surface 210 to the first set of image sensors 21 and the second optical signal 31 generated by the second surface 220 to the second set of image sensors 22.

As such, the beamsplitter assembly 50 splits the light beam from the objective lens 10 into a plurality of light beams and converges the light beams to corresponding image sensors 20 for imaging, so as to achieve simultaneous detection of different optical signals emitted from the two surfaces of the sample 200 of interest.

Specifically, the beamsplitter assembly 50 may reflect a part of the light beams according to the wavelength of the light beams and transmit another part of the light beams, or reflect a part of the light beams according to the throughput and transmit another part of the light beams.

Referring to FIG. 1, in some embodiments, the first set of image sensors 21 at least comprise a first image sensor 211 and a second image sensor 212; the first set of tube lenses 40 at least comprise a first tube lens 406 and a second tube lens 407 that are identical; the beamsplitter assembly 50 is configured for splitting the first optical signal 30 into a light beam with a first wavelength 301 and a light beam with a second wavelength 302, and simultaneously delivering the light beam with the first wavelength 301 to the first image sensor 211 through the first tube lens 406 and the light beam with the second wavelength 302 to the second image sensor 212 through the second tube lens 407.

As such, the first image sensor 211 and the second image sensor 212 can separately image, so as to detect the first optical signal 30 emitted by the first surface 210 of the sample of interest. Preferably, the first optical signal 30 comprises fluorescence signals of two wavelengths, and the first image sensor 211 and the second image sensor 212 can simultaneously image on the basis of the fluorescence signals of the two wavelengths, so as to improve the imaging efficiency for various fluorescence signals. For example, taking a sequencing chip sample 200 as an example, the first image sensor 211 can acquire the fluorescence generated by base A/G on the first surface 210 and image, and the second image sensor 212 can acquire the fluorescence generated by base C/T on the first surface 210 and image. Specifically, in one sequencing process, the first optical signal 30 comprises a light beam with a first wavelength 301 generated by base A and a light beam with a second wavelength 302 generated by base C; the first image sensor 211 is configured for acquiring the light beam with the first wavelength 301 generated by base A and imaging, and the second image sensor 212 is configured for acquiring the light beam with the second wavelength 302 generated by base C and imaging. Alternatively, in one sequencing process, the first optical signal 30 comprises a light beam with a first wavelength 301 generated by base G and a light beam with a second wavelength 302 generated by base T; the first image sensor 211 is configured for acquiring the light beam with the first wavelength 301 generated by base G and imaging, and the second image sensor 212 is configured for acquiring the light beam with the second wavelength 302 generated by base T and imaging. In this way, the fluorescence signals of two wavelengths can be simultaneously imaged by the first image sensor 211 and the second image sensor 212.

Referring to FIG. 1, in some embodiments, the second set of image sensors 22 at least comprise a third image sensor 221 and a fourth image sensor 222; the second set of tube lenses 41 at least comprise a third tube lens 416 and a fourth tube lens 417 that are identical; the beamsplitter assembly 50 is configured for splitting the second optical signal 31 into a light beam with a third wavelength 311 and a light beam with a fourth wavelength 312, and simultaneously delivering the light beam with the third wavelength 311 to the third image sensor 221 through the third tube lens 416 and the light beam with the fourth wavelength 312 to the fourth image sensor 222 through the fourth tube lens 417. As such, the third image sensor 221 and the fourth image sensor 222 can separately image, so as to detect the second optical signal 31 emitted by the second surface 220 of the sample of interest. Preferably, the second optical signal 31 comprises fluorescence signals of two wavelengths, and the third image sensor 221 and the fourth image sensor 222 can simultaneously image on the basis of the fluorescence signals of the two wavelengths, so as to improve the imaging efficiency for various fluorescence signals.

For example, taking a sequencing chip sample 200 as an example, the third image sensor 221 can acquire the fluorescence generated by base A/G on the second surface 220 and image, and the fourth image sensor 222 can acquire the fluorescence generated by base C/T on the second surface 220 and image. Specifically, in one sequencing process, the second optical signal 31 comprises a light beam with a third wavelength 311 emitted by base A and a light beam with a fourth wavelength 312 emitted by base C; the third image sensor 221 is configured for acquiring the light beam with the third wavelength 311 emitted by base A and imaging, and the fourth image sensor 222 is configured for acquiring the light beam with the fourth wavelength 312 emitted by base C and imaging. Alternatively, in one sequencing process, the second optical signal 31 comprises a light beam with a third wavelength 311 emitted by base G and a light beam with a fourth wavelength 312 emitted by base T; the third image sensor 221 is configured for acquiring the light beam with the third wavelength 311 emitted by base G and imaging, and the fourth image sensor 222 is configured for acquiring the light beam with the fourth wavelength 312 emitted by base T and imaging. In this way, the fluorescence signals of two wavelengths can be simultaneously imaged by the third image sensor 221 and the fourth image sensor 222.

In some embodiments, the wavelengths of the fluorescence generated by the same base on the first surface 210 and the second surface 220 are identical. For example, the wavelength of the fluorescence generated by base A on the first surface 210 is identical to that on the second surface 220, and the wavelength of the fluorescence generated by base T on the first surface 210 is identical to that on the second surface 220.

Referring to FIG. 1, in some embodiments, the beamsplitter assembly 50 comprises a first dichroic mirror 51, a first beamsplitter 52, and a second beamsplitter 53; the first dichroic mirror 51 is configured for reflecting the light beam with the first wavelength 301 to the first beamsplitter 52 and transmitting the light beam with the second wavelength 302 to the second beamsplitter 53; the first beamsplitter 52 is configured for receiving the light beam with the first wavelength 301 reflected from the first dichroic mirror 51 and reflecting the light beam with the first wavelength 301 to the first tube lens 406, so as to deliver the light beam with the first wavelength 301 to the first image sensor 211 through the first tube lens 406; the second beamsplitter 53 is configured for receiving the light beam with the second wavelength 302 transmitted from the first dichroic mirror 51 and reflecting the light beam with the second wavelength 302 to the second tube lens 407, so as to deliver the light beam with the second wavelength 302 to the second image sensor 212 through the second tube lens 407.

As such, the beamsplitter assembly 50 can separate optical signals with different wave bands and different throughputs, allowing the first image sensor 211 to image on the basis of the light beam with the first wavelength 301 and the second image sensor 212 to image on the basis of the light beam with the second wavelength 302 simultaneously.

Specifically, the first dichroic mirror 51 separates the first optical signal 30 into a light beam with a first wavelength 301 and a light beam with a second wavelength 302 according to the wavelength of the light beam; the first beamsplitter 52 reflects the light beam with the first wavelength 301 to the first tube lens 406 for converging according to the throughput of the light beam, and the second beamsplitter 53 reflects the light beam with the second wavelength 302 to the second tube lens 407 for converging according to the throughput of the light beam.

In some embodiments, the first dichroic mirror 51 and the first beamsplitter 52 are generally parallel, the first dichroic mirror 51 and the second beamsplitter 53 are generally perpendicular, and the first dichroic mirror 51 is placed obliquely at an included angle of 45° with respect to the optical axis of the objective lens 10, thereby turning the light beam, arranging the image sensors 20 and the objective lens 10 at different positions, and making the imaging system 100 more compact in structure.

Referring to FIG. 1, in some embodiments, the first dichroic mirror 51 is configured for reflecting the light beam with the third wavelength 311 to the first beamsplitter 52 and transmitting the light beam with the fourth wavelength 312 to the second beamsplitter 53; the first beamsplitter 52 is configured for receiving the light beam with the third wavelength 311 reflected from the first dichroic mirror 51 and transmitting the light beam with the first wavelength 311 to the third tube lens 416, so as to deliver the light beam with the third wavelength 311 to the third image sensor 221 through the third tube lens 416; the second beamsplitter 53 is configured for receiving the light beam with the fourth wavelength 312 transmitted from the first dichroic mirror 51 and transmitting the light beam with the fourth wavelength 312 to the fourth tube lens 417, so as to deliver the light beam with the fourth wavelength 312 to the fourth image sensor 222 through the fourth tube lens 417.

As such, the beamsplitter assembly 50 can separate optical signals with different wave bands and different throughputs, allowing the third image sensor 221 to image on the basis of the light beam with the third wavelength 311 and the fourth image sensor 222 to image on the basis of the light beam with the fourth wavelength 312 simultaneously.

Specifically, the first dichroic mirror 51 separates the second optical signal 31 into a light beam with a third wavelength 311 and a light beam with a fourth wavelength 312 according to the wavelength of the light beam; the first beamsplitter 52 transmits the light beam with the third wavelength 311 to the third tube lens 416 for converging according to the throughput of the light beam, and the second beamsplitter 53 transmits the light beam with the fourth wavelength 312 to the fourth tube lens 417 for converging according to the throughput of the light beam.

In some embodiments, the beamsplitter assembly 50 further comprises a mirror 54 configured for reflecting the light beam from the objective lens 10 to the first dichroic mirror 51. The mirror 54 is arranged on the optical axis of the objective lens 10 and is generally parallel with the first dichroic mirror 51.

Referring to FIG. 1, in some embodiments, the imaging system 100 comprises a light source 60 and a second dichroic mirror 61 arranged between the light source 60 and the objective lens 10, wherein the second dichroic mirror 61 is arranged on the optical axis of the objective lens 10, the light source 60 is configured for emitting an excitation light beam, and the second dichroic mirror 61 is configured for reflecting the excitation light beam toward the objective lens 10, such that the excitation light beam is projected through the objective lens 10 to the first surface 210 and the second surface 220.

As such, the light source 60 can excite the bases with an optically detectable label binding to the biomolecule to generate an optical signal, thus achieving the base sequence determination of the biomolecule.

Specifically, the light source 60 can emit a light beam with at least one wavelength to excite fluorophores on more than one base, so as to achieve the sequencing of multiple bases. Illustratively, the light source 60 may excite the fluorescence signal of base A and the fluorescence signal of base C simultaneously, or may excite the fluorescence signal of base G and the fluorescence signal of base T simultaneously. In some embodiments, the second dichroic mirror 61 may be arranged in parallel with the mirror 54.

Referring to FIG. 1, in some embodiments, the imaging system 100 comprises a filter 62. The filter 62 is arranged on the side of the first set of tube lenses 40 and the second set of tube lenses 41 distal to the image sensors 20, and can filter light beams, allow the fluorescence waveband to pass through, and cut off the undesired excitation waveband, thereby improving the imaging quality of the image sensors 20.

Referring to FIG. 1, in some embodiments, the imaging system 100 comprises an autofocus apparatus 70, wherein the autofocus apparatus 70 is configured for detecting the positional relationship between the sample 200 of interest and the objective lens 10 and for positioning the sample 200 of interest in the focal plane of the objective lens 10 according to the detected relative positions of the sample 200 of interest and the objective lens 10.

As such, the imaging system 100 can always guarantee sharp focusing and ensure the accuracy of acquired images.

Specifically, the autofocus apparatus 70 is mainly configured for marking the object distance after the objective lens 10 finishes the focusing, monitoring the variation of the object distance in real time during the gene sequencing process, and correcting the variation, so as to ensure that the imaging system 100 always focuses sharply, ensure the accuracy of the images acquired by the image sensors 20, and finally give sharp images within the depth of focus range. When the autofocus apparatus 70 focuses on and tracks the first surface 210, the variation in the thickness of the chip substrate will allocate a part of the depth of field of the second surface 220. Referring to FIG. 5, the change profiles of the depths of focus of the first surface 210 and the second surface 220 are consistent, and the field curves on the first surface 210 and the second surface 220 are approximately coincident, suggesting that the depths of focus and the depths of field of the first surface 210 and the second surface 220 are also consistent. When focusing, the system just needs to complete the focusing on one of the first surface 210 and the second surface 220, and the focusing on the other surface will be completed automatically.

In some embodiments, the objective lens 10 has a numerical aperture greater than 0.6 and a field of view greater than 1.2 mm.

The aperture and the field of view are beneficial to detecting a greater area on the sample, thus allowing the system to provide more comprehensive and detailed measurement results and more information. As such, the imaging system 100 may fit a greater range of optical devices.

It will be appreciated that the field of view may be the diameter of the circular area actually and effectively viewable on the sample of interest under the objective lens 10. In some embodiments, the numerical aperture of the objective lens 10 may be 0.8, where the objective lens 10 is available without regard to an optimization design. A greater numerical aperture of the objective lens 10 will lead to higher resolutions of the first set of tube lenses 40 and the second set of tube lenses 41 and smaller first depth of field and second depth of field.

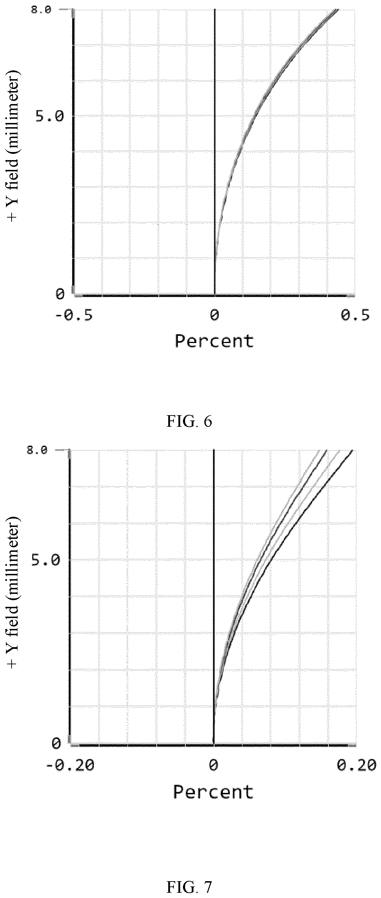

Referring to FIG. 6, in some embodiments, the combination of the objective lens 10 and the first set of tube lenses 40 has a distortion of less than 0.5.

As such, the image generated by the first set of image sensors 21 is less distorted, thereby ensuring the accuracy of the sequencing on the first surface 210.

Specifically, the distortion is the deformation of an image, and generally includes barrel distortion and pincushion distortion. The real scene of the object side is deformed on the image surface after passing through the imaging system 100, and the distortion does not influence the definition of images. Optics manufacturers have specialized instruments and equipment for measuring distortion.

Referring to FIG. 7, in some embodiments, the combination of the objective lens 10 and the second set of tube lenses 41 has a distortion of less than 0.2.

As such, the image generated by the second set of image sensors 22 is less distorted, thereby ensuring the accuracy of the sequencing on the second surface 220.

Specifically, the factors affecting the distortion include wavelength size, magnification, field size, and the like.

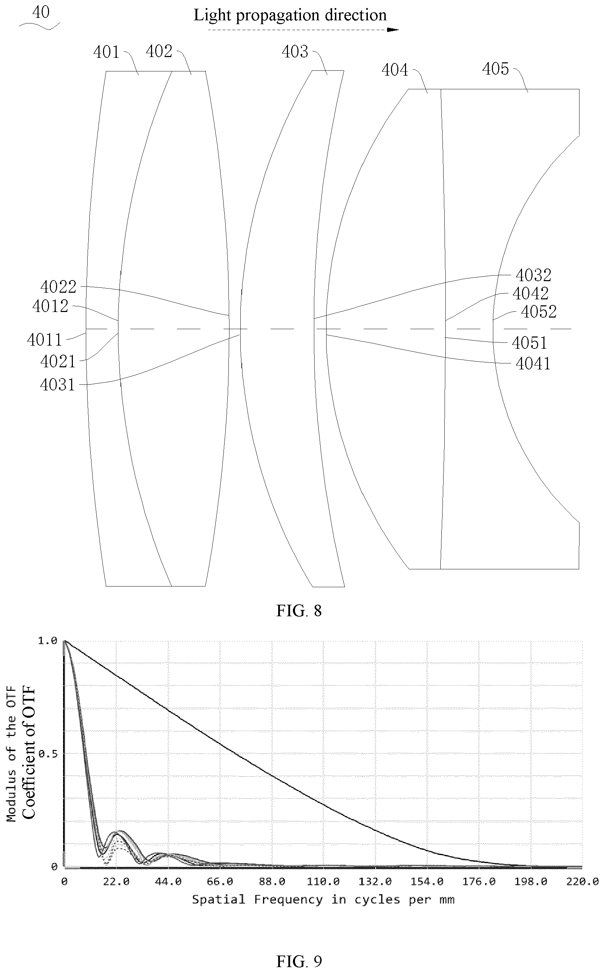

Referring to FIG. 8, in some embodiments, the first set of tube lenses 40 comprise a first optical lens 401, a second optical lens 402, a third optical lens 403, a fourth optical lens 404, and a fifth optical lens 405 in sequence, wherein the first optical lens 401 has a negative refractive power, the second optical lens 402 has a positive refractive power, the third optical lens 403 has a positive refractive power, the fourth optical lens 404 has a positive refractive power, and the fifth optical lens 405 has a negative refractive power; the object-side surfaces and the image-side surfaces of the first optical lens 401, the second optical lens 402, the third optical lens 403, the fourth optical lens 404, and the fifth optical lens 405 are all spherical surfaces, and the object-side surface of one optical lens and the image-side surface of the other optical lens in each pair of adjacent optical lenses are arranged towards each other.

According to embodiments of the present application, the first set of tube lenses 40 comprise five optical lenses and thus feature compactness; the object-side surfaces and the image-side surfaces of the five optical lenses are all spherical surfaces, allowing the first set of tube lenses 40 to greatly reduce the aberration in a visible wavelength range of 553 nm to 712 nm. Compared with aspherical optical lenses, the optical lenses according to embodiments of the present application feature ease to manufacture, and thereby provide the first set of tube lenses 40 ease to manufacture. The combination of the first set of tube lenses 40 with an infinite conjugate objective lens 10 can sharply converge base fluorescence spots from the first surface 210 on an image surface of the first set of image sensors 21. Specifically, the first optical lens 401, the second optical lens 402, the third optical lens 403, the fourth optical lens 404, and the fifth optical lens 405 are arranged in sequence along the light propagation direction (as indicated by the dashed arrow in FIG. 8).

The aforementioned "positive refractive power" indicates that the optical lens has a positive focal length and a light-converging effect. The "negative refractive power" indicates that the optical lens has a negative focal length and a light-diverging effect.

Taking the optical lens as a boundary, the side where the object is located is the object side, and the surface of the optical lens proximal to the object side is referred to as the object-side surface; the side where the image of the object is located is the image side, and the surface of the optical lens proximal to the image side is referred to as the image-side surface.

Referring to FIG. 8, in some embodiments, the object-side surface 4011 of the first optical lens 401 is convex at the optical axis of the first optical lens 401, and the image-side surface 4012 of the first optical lens 401 is concave at the optical axis of the first optical lens 401; the object-side surface 4011 of the first optical lens 401 has a curvature radius of 160 mm to 190 mm at the optical axis of the first optical lens 401, and the image-side surface 4012 of the first optical lens 401 has a curvature radius of 60 mm to 90 mm at the optical axis of the first optical lens 401.

When the first optical lens 401 meets the curvature radius, the design difficulty and the assembly sensitivity of the first set of tube lenses 40 can be reduced.

The positivity or negativity of the curvature radius indicates that the optical surface is convex towards the object side or convex towards the image side. When an optical surface (including the object-side surface and the image-side surface) is convex towards the object side, the curvature radius of the optical surface is a positive value; when an optical surface (including the object-side surface and the image-side surface) is convex toward the image side, the optical surface is concave toward the object side, and the curvature radius of the optical surface is a negative value. That is, the curvature radius of the object-side surface 4011 of the first optical lens 401 may be any value in interval [160 mm, 190 mm]; for example, the value may be 160, 170, 180, 190, etc., in mm. The curvature radius of the image-side surface 4012 of the first optical lens 401 may be any value in interval [60 mm, 90 mm]; for example, the value may be 60, 70, 80, 90, etc., in mm.

Referring to FIG. 8, in some embodiments, the object-side surface 4021 of the second optical lens 402 is convex at the optical axis of the second optical lens 402, and the image-side surface 4022 of the second optical lens 402 is convex at the optical axis of the second optical lens 402; the object-side surface 4021 of the second optical lens 402 has a curvature radius of 60 mm to 90 mm at the optical axis of the second optical lens 402, and the image-side surface 4022 of the second optical lens 402 has a curvature radius of -170 mm to -140 mm at the optical axis of the second optical lens 402.

When the second optical lens 402 meets the curvature radius, the design difficulty and the assembly sensitivity of the first set of tube lenses 40 can be reduced.

That is, the curvature radius of the object-side surface 4021 of the second optical lens 402 may be any value in interval [60 mm, 90 mm]; for example, the value may be 60, 70, 80, 90, etc., in mm. The curvature radius of the image-side surface 4022 of the second optical lens 402 may be any value in interval [-170 mm, -140 mm]; for example, the value maybe -170, -160, -150, -140, etc., in mm.

Referring to FIG. 8, in some embodiments, the object-side surface 4031 of the third optical lens 403 is convex at the optical axis of the third optical lens 403, and the image-side surface 4032 of the third optical lens 403 is concave at the optical axis of the third optical lens 403; the object-side surface 4031 of the third optical lens 403 has a curvature radius of 40 mm to 70 mm at the optical axis of the third optical lens 403, and the image-side surface 4032 of the third optical lens 403 has a curvature radius of 110 mm to 140 mm at the optical axis of the third optical lens 403.

When the third optical lens 403 meets the curvature radius, the design difficulty and the assembly sensitivity of the first set of tube lenses 40 can be reduced.

That is, the curvature radius of the object-side surface 4031 of the third optical lens 403 may be any value in interval [40 mm, 70 mm]; for example, the value may be 40, 50, 60, 70, etc., in mm. The curvature radius of the image-side surface 4032 of the third optical lens 403 may be any value in interval [110 mm, 140 mm]; for example, the value may be 110, 120, 130, 140, etc., in mm.

Referring to FIG. 8, in some embodiments, the object-side surface 4041 of the fourth optical lens 404 is convex at the optical axis of the fourth optical lens 404, and the image-side surface 4042 of the fourth optical lens 404 is convex at the optical axis of the fourth optical lens 404; the object-side surface 4041 of the fourth optical lens 404 has a curvature radius of 30 mm to 50 mm at the optical axis of the fourth optical lens 404, and the image-side surface 4042 of the fourth optical lens 404 has a curvature radius of -700 mm to -650 mm at the optical axis of the fourth optical lens 404.

When the fourth optical lens 404 meets the curvature radius, the design difficulty and the assembly sensitivity of the first set of tube lenses 40 can be reduced.

That is, the curvature radius of the object-side surface 4041 of the fourth optical lens 404 may be any value in interval [30 mm, 50 mm]; for example, the value may be 30, 35, 40, 45, 50, etc., in mm. The curvature radius of the image-side surface 4042 of the fourth optical lens 404 may be any value in interval [-700 mm, -650 mm]; for example, the value may be -700, -690, -680, -670, -660, -650, etc., in mm.

Referring to FIG. 8, in some embodiments, the object-side surface 4051 of the fifth optical lens 405 is concave at the optical axis of the fifth optical lens 405, and the image-side surface 4052 of the fifth optical lens 405 is concave at the optical axis of the fifth optical lens 405; the object-side surface 4051 of the fifth optical lens 405 has a curvature radius of -700 mm to -650 mm at the optical axis of the fifth optical lens 405, and the image-side surface 4052 of the fifth optical lens 405 has a curvature radius of 20 mm to 40 mm at the optical axis of the fifth optical lens 405.

When the fifth optical lens 405 meets the curvature radius, the design difficulty and the assembly sensitivity of the first set of tube lenses 40 can be reduced.

That is, the curvature radius of the object-side surface 4051 of the fifth optical lens 405 may be any value in interval [-700 mm, -650 mm]; for example, the value may be -700, -690, -680, -670, -660, -650, etc., in mm. The curvature radius of the image-side surface 4052 of the fifth optical lens 405 may be any value in interval [20 mm, 40 mm]; for example, the value may be 20, 25, 30, 35, 40, etc., in mm.

In some embodiments, the first optical lens 401 has a thickness of 2 mm to 5 mm at the optical axis of the first optical lens 401, the second optical lens 402 has a thickness of 9 mm to 13 mm at the optical axis of the second optical lens 402, the third optical lens 403 has a thickness of 6 mm to 10 mm at the optical axis of the third optical lens 403, the fourth optical lens 404 has a thickness of 9 mm to 14 mm at the optical axis of the fourth optical lens 404, and the fifth optical lens 405 has a thickness of 3 mm to 7 mm at the optical axis of the fifth optical lens 405.

Through the proper configuration of the thicknesses of different optical lenses, the optimal balance between the miniaturization of the first set of tube lenses 40 and the manufacturability of the optical lenses may be achieved, thereby avoiding compromised strength of the first set of tube lenses 40 due to excessively thin optical lenses and thus affected fabrication yield. In addition, when the thicknesses of the optical lenses at their optical axes are in the above ranges, the first set of tube lenses 40 can greatly reduce the aberration in a visible wavelength range of 553 nm to 712 nm. The combination of the first set of tube lenses 40 with an infinite conjugate objective lens 10 can sharply converge base fluorescence spots from the first surface 210 on an image surface of the first set of image sensors 21. That is, the thickness of the first optical lens 401 at the optical axis of the first optical lens 401 may be any value in interval [2.0 mm, 5.0 mm]; for example, the value may be 2.0, 2.5, 3.0, 3.5, 4.0, 4.5, 5.0, etc., in mm.

The thickness of the second optical lens 402 at the optical axis of the second optical lens 402 may be any value in interval [9.0 mm, 13.0 mm]; for example, the value may be 9.0, 10.0, 11.0, 12.0, 13.0, etc., in mm.

The thickness of the third optical lens 403 at the optical axis of the third optical lens 403 may be any value in interval [6.0 mm, 10.0 mm]; for example, the value may be 6.0, 7.0, 8.0, 9.0, 10.0, etc., in mm.

The thickness of the fourth optical lens 404 at the optical axis of the fourth optical lens 404 may be any value in interval [9.0 mm, 14.0 mm]; for example, the value may be 9.0, 10.0, 11.0, 12.0, 13.0, 14.0, etc., in mm.

The thickness of the fifth optical lens 405 at the optical axis of the fifth optical lens 405 may be any value in interval [3.0 mm, 7.0 mm]; for example, the value may be 3.0, 4.0, 5.0, 6.0, 7.0, etc., in mm.

In some embodiments, the first optical lens 401 and the second optical lens 402 are cemented to form an optical lens set, and the fourth optical lens 404 and the fifth optical lens 405 are cemented to form an optical lens set.

Through the cemented optical lenses, the optical axes of the first optical lens 401 and the second optical lens 402 are aligned, and the optical axes of the fourth optical lens 404 and the fifth optical lens 405 are aligned, thus eliminating the chromatic aberration between the first optical lens 401 and the second optical lens 402 and the chromatic aberration between the fourth optical lens 404 and the fifth optical lens 405, and allowing the first set of tube lenses 40 to greatly reduce the aberration in a visible wavelength range of 553 nm to 712 nm. The combination of the first set of tube lenses 40 with an infinite conjugate objective lens 10 can sharply converge the optical signals from the first surface 210 on an image surface of the first set of image sensors 21. The cemented optical lens is also beneficial to the assembly and calibration of the first set of tube lenses 40.

In the manufacturing process of the optical lenses, two optical lenses are usually cemented together by resin cementation or optical cementation, and the cement is cured to fix the optical lenses, so as to give cemented optical lens sets.

Specifically, the first optical lens 401, the second optical lens 402, the third optical lens 403, the fourth optical lens 404, and the fifth optical lens 405 may be made of different materials. Additionally, the first optical lens 401 may be made of flint glass, the second optical lens 402 may be made of crown glass, the third optical lens 403 may be made of crown glass, the fourth optical lens 404 may be made of flint glass, and the fifth optical lens 405 may be made of crown glass.

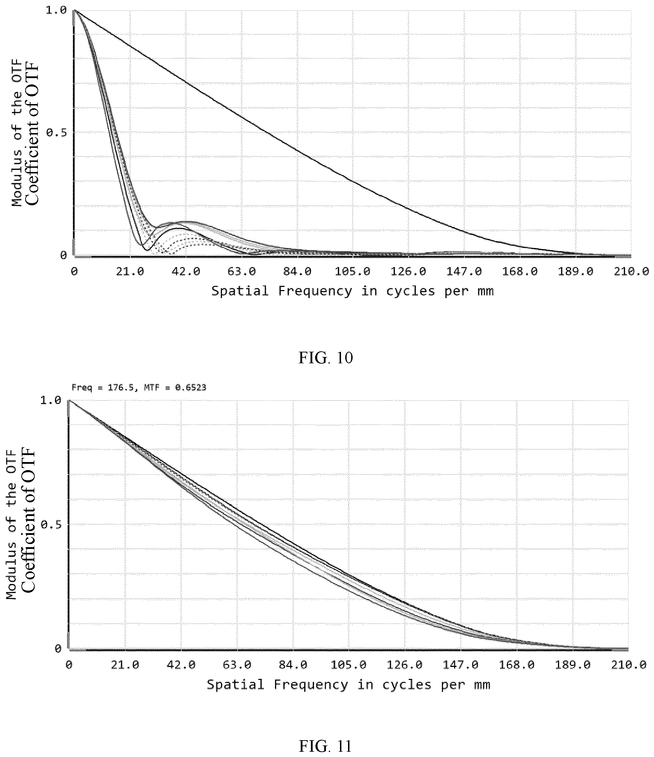

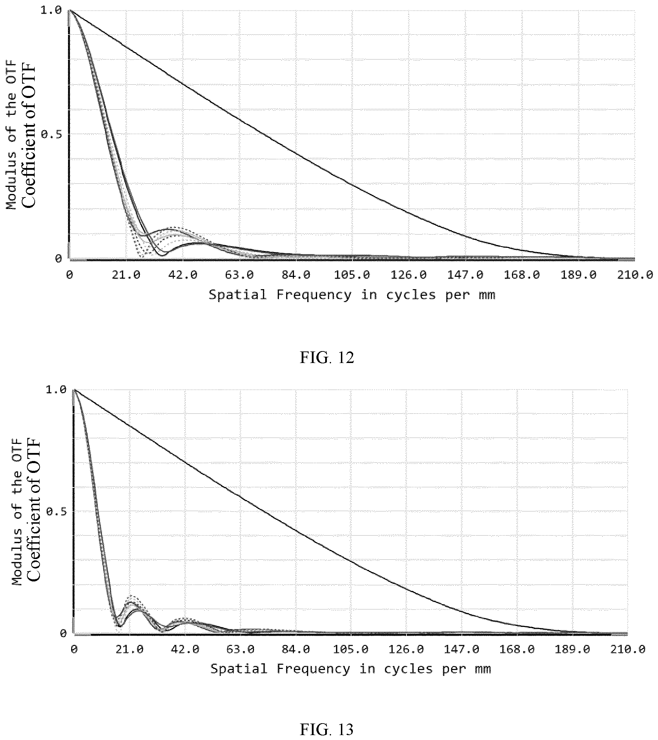

FIGs. 9 to 13 are MTF curves of the combination of the first set of tube lenses 40 with the objective lens 10 when the thickness of the first liquid layer 202 is 45 $\mu$m, 47 $\mu$m, 50 $\mu$m, 53 $\mu$m, or 55 $\mu$m, respectively. The MTF curves can be used to evaluate the resolution indicators of the first set of tube lenses 40. In the figures, the abscissa represents the spatial frequency, and the ordinate represents the value of MTF. The maximal value is 1, and a greater value indicates a higher resolution. As can be seen, when the thickness of the first liquid layer 202 is 50 $\mu$m, the MTF curves of the combination of the first set of tube lenses 40 with the objective lens 10 in different fields of view are all close to the diffraction limit at different spatial frequencies, and the optimal resolution is achieved.

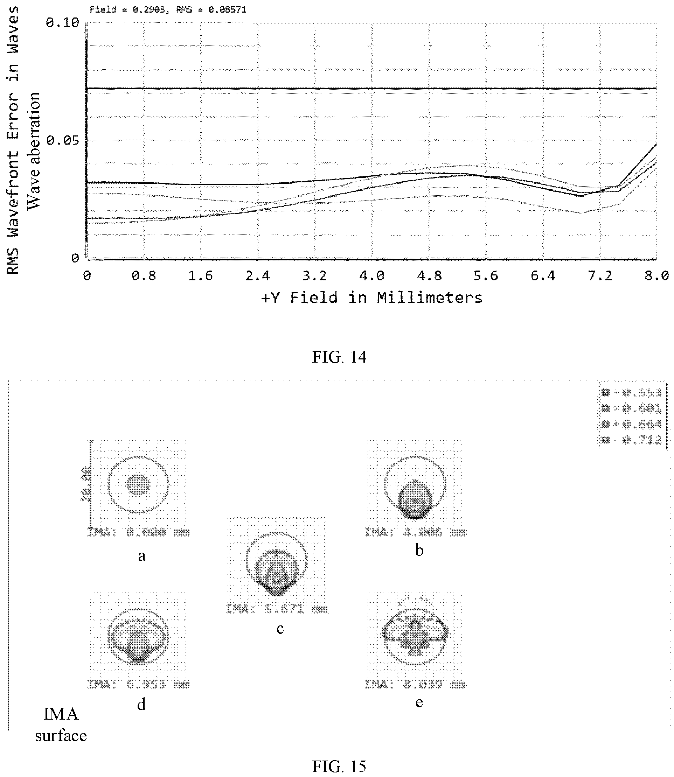

FIG. 14 is a diagram illustrating the wavefront aberration curves of the combination of the first set of tube lenses 40 with the objective lens 10. The wavefront aberration curve is also an indicator for evaluating the image quality of the imaging system 100, and a higher MTF value generally indicates a better wavefront aberration curve. As shown in

FIG. 14, after the first set of tube lenses 40 is combined with the objective lens 10, the wavefront aberrations in different fields of view are lower than the diffraction limit, and the curves are relatively concentrated, achieving a better image quality.

FIG. 15 is a spot diagram of the combination of the first set of tube lenses 40 with the objective lens 10 in 5 different fields of view. The spot diagram is a spot pattern observed at the image side of the spots in the field of view at the object side. The spot diagram has a diffraction-limited circle with an airy radius of 6.369 μm, and the root mean square (RMS) radii of the spots in the fields of view coded a-e are 1.242 μm, 3.485 μm, 3.577 μm, 2.876 μm, and 3.058 μm, respectively. It can be seen that, in the first set of tube lenses 40 according to embodiments of the present application, the spots of the 5 fields of view are all within or close to the diffraction-limited circle. The spot size of the first set of tube lenses 40 reaches the diffraction limit in the fields of view, and a better image quality is achieved, suggesting good aberration of the imaging system 100 comprising the first set of tube lenses 40.

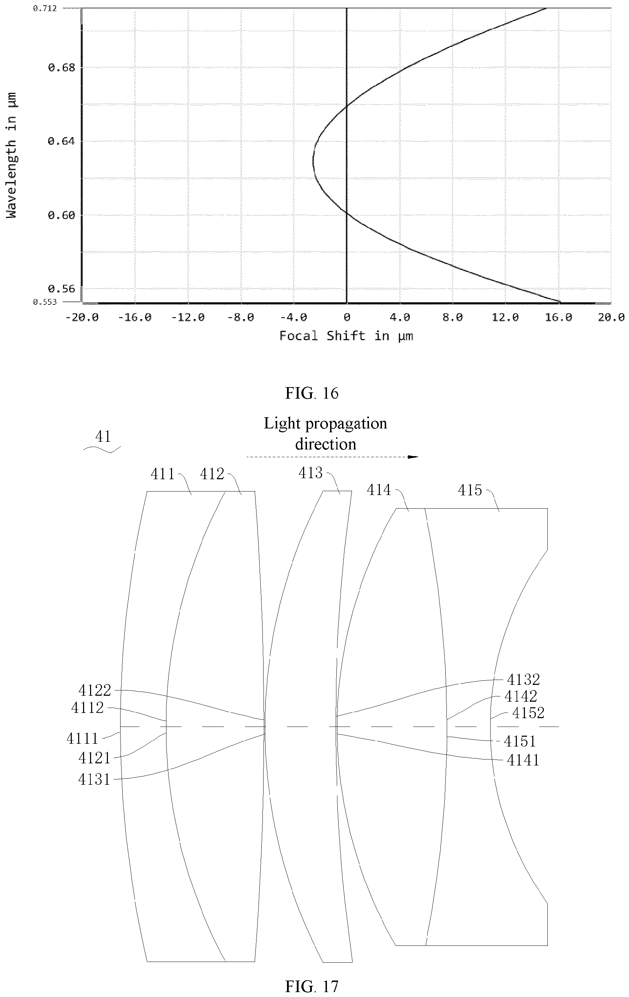

FIG. 16 is a diagram illustrating the focal shift curve of the combination of the first set of tube lenses 40 with the objective lens 10. The focal shift diagram is a positional diagram of the focuses of light beams of different wavelengths. It can be seen from the figure that the maximum focal shift range, or axial chromatic aberration, from 553 nm to 712 nm is 18.7199 μm.

Referring to FIG. 17, in some embodiments, the second set of tube lenses 41 comprise a sixth optical lens 411, a seventh optical lens 412, an eighth optical lens 413, a ninth optical lens 414, and a tenth optical lens 415 in sequence, wherein the sixth optical lens 411 has a negative refractive power, the seventh optical lens 412 has a positive refractive power, the eighth optical lens 413 has a positive refractive power, the ninth optical lens 414 has a positive refractive power, and the tenth optical lens 415 has a negative refractive power; the object-side surfaces and the image-side surfaces of the sixth optical lens 411, the seventh optical lens 412, the eighth optical lens 413, the ninth optical lens 414, and the tenth optical lens 415 are all spherical surfaces, and the object-side surface of one lens and the image-side surface of the other lens in each pair of adjacent lenses are arranged towards each other.

According to embodiments of the present application, the second set of tube lenses 41 comprise five lenses and thus feature compactness; the object-side surfaces and the image-side surfaces of the five lenses are all spherical surfaces, allowing the second set of tube lenses 41 to greatly reduce the aberration in a visible wavelength range of 553 nm to 712 nm. Compared with aspherical lenses, the lenses according to embodiments of the present application feature ease to manufacture, and thereby provide the second set of tube lenses 41 ease to manufacture. The combination of the second set of tube lenses 41 with an infinite conjugate objective lens 10 can sharply converge optical signals from the second surface 220 on an image surface of the second set of image sensors 22.

Specifically, the sixth optical lens 411, the seventh optical lens 412, the eighth optical lens 413, the ninth optical lens 414, and the tenth optical lens 415 are arranged in sequence along the light propagation direction (as indicated by the dashed arrow in FIG. 17).

The aforementioned "positive refractive power" indicates that the lens has a positive focal length and a light-converging effect. The "negative refractive power" indicates that the lens has a negative focal length and a light-diverging effect.

Taking the lens as a boundary, the side where the object is located is the object side, and the surface of the lens proximal to the object side is referred to as the object-side surface; the side where the image of the object is located is the image side, and the surface of the lens proximal to the image side is referred to as the image-side surface. Referring to FIG. 17, in some embodiments, the object-side surface 4111 of the sixth optical lens 411 is convex at the optical axis of the sixth optical lens 411, and the image-side surface 4112 of the sixth optical lens 411 is concave at the optical axis of the sixth optical lens 411; the object-side surface 4111 of the sixth optical lens 411 has a curvature radius of 110 mm to 130 mm at the optical axis of the sixth optical lens 411, and the image-side surface 4112 of the sixth optical lens 411 has a curvature radius of 40 mm to 70 mm at the optical axis of the sixth optical lens 411. When the sixth optical lens 411 meets the curvature radius, the design difficulty and the assembly sensitivity of the second set of tube lenses 41 can be reduced.

The positivity or negativity of the curvature radius indicates that the optical surface is convex towards the object side or convex towards the image side. When an optical surface (including the object-side surface and the image-side surface) is convex towards the object side, the curvature radius of the optical surface is a positive value; when an optical surface (including the object-side surface and the image-side surface) is convex toward the image side, the optical surface is concave toward the object side, and the curvature radius of the optical surface is a negative value. That is, the curvature radius of the object-side surface 4111 of the sixth optical lens 411 may be any value in interval [110 mm, 130 mm]; for example, the value may be 110, 115, 120, 125, 130, etc., in mm. The curvature radius of the image-side surface 4112 of the sixth optical lens 411 may be any value in interval [40 mm, 70 mm]; for example, the value may be 40, 50, 60, 70, etc., in mm.

Referring to FIG. 17, in some embodiments, the object-side surface 4121 of the seventh optical lens 412 is convex at the optical axis of the seventh optical lens 412, and the image-side surface 4122 of the seventh optical lens 412 is convex at the optical axis of the seventh optical lens 412; the object-side surface 4121 of the seventh optical lens 412 has a curvature radius of 40 mm to 70 mm at the optical axis of the seventh optical lens 412, and the image-side surface 4122 of the seventh optical lens 412 has a curvature radius of -350 mm to -300 mm at the optical axis of the seventh optical lens 412.

When the seventh optical lens 412 meets the curvature radius, the design difficulty and the assembly sensitivity of the second set of tube lenses 41 can be reduced.

That is, the curvature radius of the object-side surface 4121 of the seventh optical lens 412 may be any value in interval [40 mm, 70 mm]; for example, the value may be 40, 50, 60, 70, etc., in mm. The curvature radius of the image-side surface 4122 of the seventh optical lens 412 may be any value in interval [-350 mm, -300 mm]; for example, the value may be -350, -340, -330, -320, -310, -300, etc., in mm.

Referring to FIG. 17, in some embodiments, the object-side surface 4131 of the eighth optical lens 413 is convex at the optical axis of the eighth optical lens 413, and the image-side surface 4132 of the eighth optical lens 413 is concave at the optical axis of the eighth optical lens 413; the object-side surface 4131 of the eighth optical lens 413 has a curvature radius of 50 mm to 70 mm at the optical axis of the eighth optical lens 413, and the image-side surface 4132 of the eighth optical lens 413 has a curvature radius of 180 mm to 220 mm at the optical axis of the eighth optical lens 413.

When the eighth optical lens 413 meets the curvature radius, the design difficulty and the assembly sensitivity of the second set of tube lenses 41 can be reduced.

That is, the curvature radius of the object-side surface 4131 of the eighth optical lens 413 may be any value in interval [50 mm, 70 mm]; for example, the value may be 50, 60, 70, etc., in mm. The curvature radius of the image-side surface 4132 of the eighth optical lens 413 may be any value in interval [180 mm, 220 mm]; for example, the value may be 180, 190, 200, 210, 220, etc., in mm.

Referring to FIG. 17, in some embodiments, the object-side surface 4141 of the ninth optical lens 414 is convex at the optical axis of the ninth optical lens 414, and the image-side surface 4142 of the ninth optical lens 414 is convex at the optical axis of the ninth optical lens 414; the object-side surface 4141 of the ninth optical lens 414 has a curvature radius of 40 mm to 60 mm at the optical axis of the ninth optical lens 414, and the image-side surface 4142 of the ninth optical lens 414 has a curvature radius of -150 mm to -110 mm at the optical axis of the ninth optical lens 414. When the ninth optical lens 414 meets the curvature radius, the design difficulty and the assembly sensitivity of the second set of tube lenses 41 can be reduced.

That is, the curvature radius of the object-side surface 4141 of the ninth optical lens 414 may be any value in interval [40 mm, 60 mm]; for example, the value may be 40, 50, 60, etc., in mm. The curvature radius of the image-side surface 4142 of the ninth optical lens 414 may be any value in interval [-150 mm, -110 mm]; for example, the value may be -150, -140, -130, -120, -110, etc., in mm.

Referring to FIG. 17, in some embodiments, the object-side surface 4151 of the tenth optical lens 415 is concave at the optical axis of the tenth optical lens 415, and the image-side surface 4152 of the tenth optical lens 415 is concave at the optical axis of the tenth optical lens 415; the object-side surface 4151 of the tenth optical lens 415 has a curvature radius of -150 mm to -110 mm at the optical axis of the tenth optical lens 415, and the image-side surface 4152 of the tenth optical lens 415 has a curvature radius of 20 mm to 50 mm at the optical axis of the tenth optical lens 415. When the tenth optical lens 415 meets the curvature radius, the design difficulty and the assembly sensitivity of the second set of tube lenses 41 can be reduced.

That is, the curvature radius of the object-side surface 4151 of the tenth optical lens 415 may be any value in interval [-150 mm, -110 mm]; for example, the value may be -150, -140, -130, -120, -110, etc., in mm. The curvature radius of the image-side surface 4152 of the tenth optical lens 415 may be any value in interval [20 mm, 50 mm]; for example, the value may be 20, 30, 40, 50, etc., in mm.

In some embodiments, the sixth optical lens 411 has a thickness of 3 mm to 7 mm at the optical axis of the sixth optical lens 411, the seventh optical lens 412 has a thickness of 9 mm to 13 mm at the optical axis of the seventh optical lens 412, the eighth optical lens 413 has a thickness of 7 mm to 10 mm at the optical axis of the eighth optical lens 413, the ninth optical lens 414 has a thickness of 10 mm to 15 mm at the optical axis of the ninth optical lens 414, and the tenth optical lens 415 has a thickness of 4 mm to 7 mm at the optical axis of the tenth optical lens 415.

Through the proper configuration of the thicknesses of different lenses, the optimal balance between the miniaturization of the second set of tube lenses 41 and the manufacturability of the lenses may be achieved, thereby avoiding compromised strength of the second set of tube lenses 41 due to excessively thin lenses and thus affected fabrication yield. In addition, when the thicknesses of the lenses at their optical axes are in the above ranges, the second set of tube lenses 41 can greatly reduce the aberration in a visible wavelength range of 553 nm to 712 nm. The combination of the second set of tube lenses 41 with an infinite conjugate objective lens 10 can sharply converge optical signals from the second surface 220 on an image surface of the second set of image sensors 22. That is, the thickness of the sixth optical lens 411 at the optical axis of the sixth optical lens 411 may be any value in interval [3.0 mm, 7.0 mm]; for example, the value may be 3.0, 4.0, 5.0, 6.0, 7.0, etc., in mm.

The thickness of the seventh optical lens 412 at the optical axis of the seventh optical lens 412 may be any value in interval [9.0 mm, 13.0 mm]; for example, the value may be 9.0, 10.0, 11.0, 12.0, 13.0, etc., in mm.

The thickness of the eighth optical lens 413 at the optical axis of the eighth optical lens 413 may be any value in interval [7.0 mm, 10.0 mm]; for example, the value may be 7.0, 7.5, 8.0, 8.5, 9.0, 9.5, 10.0, etc., in mm.

The thickness of the ninth optical lens 414 at the optical axis of the ninth optical lens 414 may be any value in interval [10.0 mm, 15.0 mm]; for example, the value may be 10.0, 11.0, 12.0, 13.0, 14.0, 15.0, etc., in mm.

The thickness of the tenth optical lens 415 at the optical axis of the tenth optical lens 415 may be any value in interval [4.0 mm, 7.0 mm]; for example, the value may be 4.5, 5.0, 5.5, 6.0, 6.5, etc., in mm.

In some embodiments, the sixth optical lens 411 and the seventh optical lens 412 are cemented to form an optical lens set, and the ninth optical lens 414 and the tenth optical lens 415 are cemented to form an optical lens set.

Through the cemented lenses, the optical axes of the sixth optical lens 411 and the seventh optical lens 412 are aligned, and the optical axes of the ninth optical lens 414 and the tenth optical lens 415 are aligned, thus eliminating the chromatic aberration between the sixth optical lens 411 and the seventh optical lens 412 and the chromatic aberration between the ninth optical lens 414 and the tenth optical lens 415, and allowing the second set of tube lenses 41 to greatly reduce the aberration in a visible wavelength range of 553 nm to 712 nm. The combination of the second set of tube lenses 41 with an infinite conjugate objective lens 10 can sharply converge the optical signals from the second surface 220 on an image surface of the second set of image sensors 22. The cemented lens is also beneficial to the assembly and calibration of the second set of tube lenses 41.

In the manufacturing process of the lenses, two lenses are usually cemented together by resin cementation or optical cementation, and the cement is cured to fix the lenses, so as to give cemented optical lens sets.

Specifically, the sixth optical lens 411, the seventh optical lens 412, the eighth optical lens 413, the ninth optical lens 414, and the tenth optical lens 415 may be made of different materials. Additionally, the sixth optical lens 411 may be made of flint glass, the seventh optical lens 412 may be made of crown glass, the eighth optical lens 413 may be made of crown glass, the ninth optical lens 414 may be made of flint glass, and the tenth optical lens 415 may be made of crown glass.

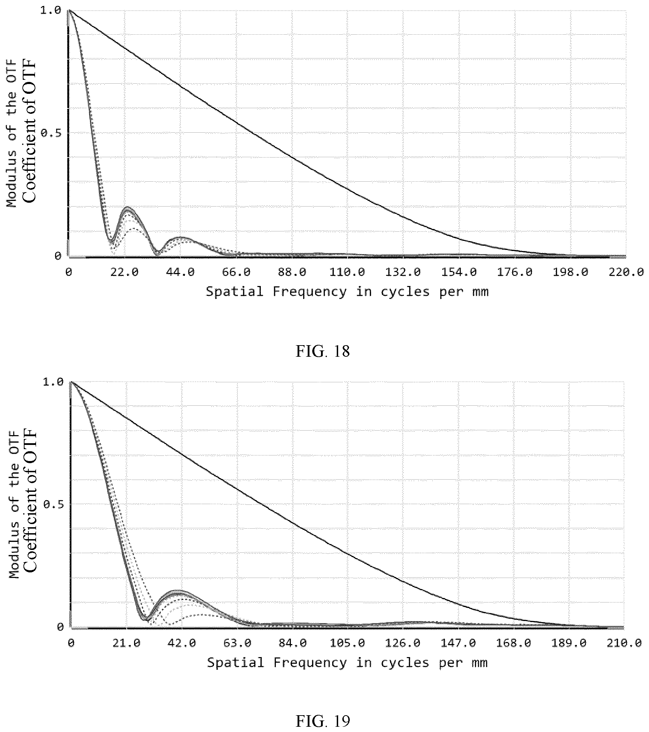

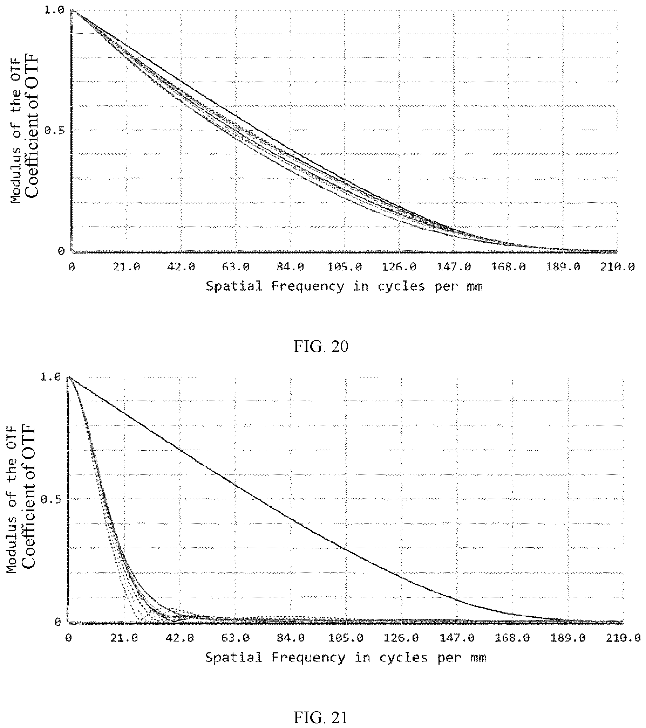

FIGs. 18 to 22 are MTF curves of the combination of the second set of tube lenses 41 with the objective lens 10 when the thickness of the first liquid layer 202 is 45 μm, 47 μm, 50 μm, 53 μm, or 55 μm, respectively. In the figures, the abscissa represents the spatial frequency, and the ordinate represents the value of MTF. The maximal value is 1, and a greater value indicates a higher resolution. As can be seen, when the thickness of the first liquid layer 202 is 50 μm, the MTF curves of the combination of the second set of tube lenses 41 with the objective lens 10 in different fields of view are all close to the diffraction limit at different spatial frequencies, and the optimal resolution is achieved.

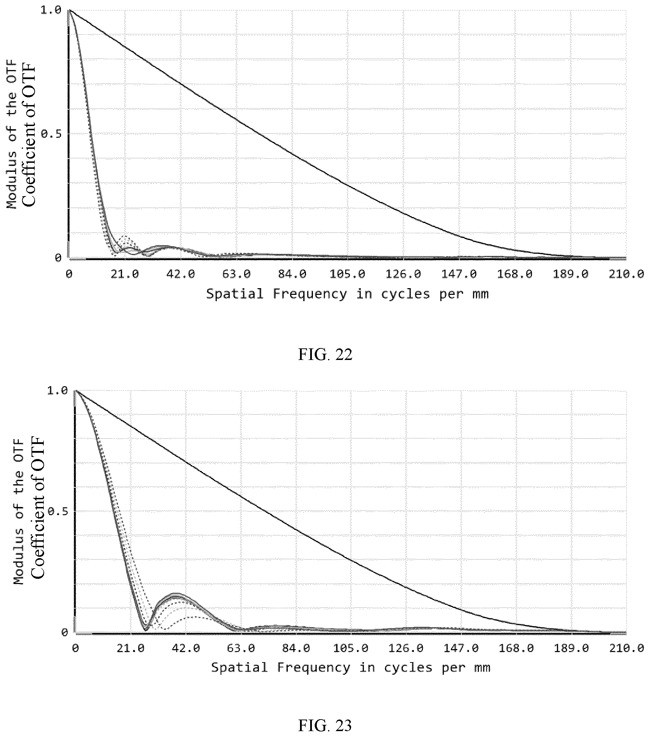

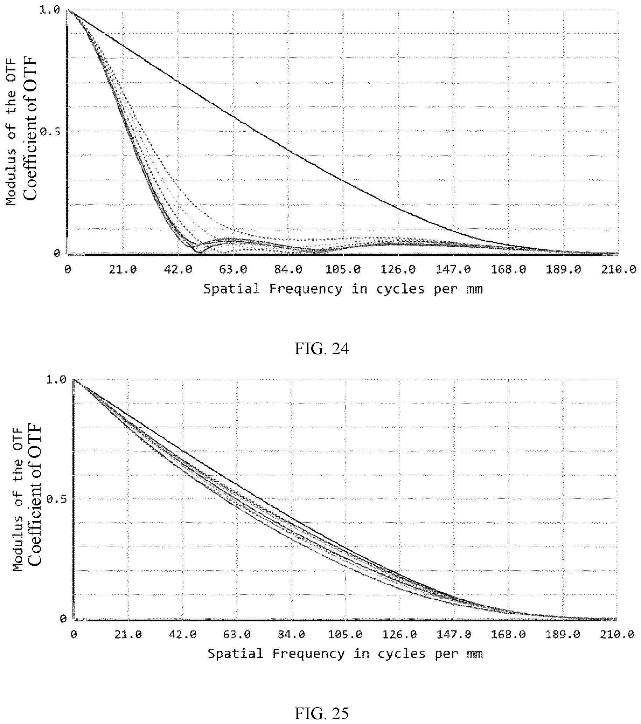

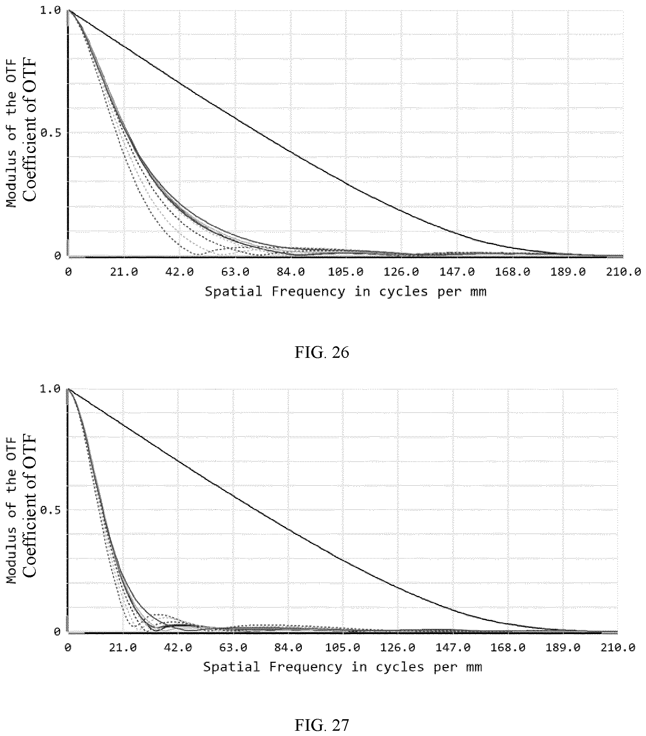

FIGs. 23 to 27 are MTF curves of the combination of the second set of tube lenses 41 with the objective lens 10 when the thickness of the second substrate 203 is 45 μm, 47 μm, 50 μm, 53 μm, or 55 μm, respectively. In the figures, the abscissa represents the spatial frequency, and the ordinate represents the value of MTF. The maximal value is 1, and a greater value indicates a higher resolution. As can be seen, when the thickness of the second substrate 203 is 50 μm, the MTF curves of the combination of the second set of tube lenses 41 with the objective lens 10 in different fields of view are all close to the diffraction limit at different spatial frequencies, and the optimal resolution is achieved.

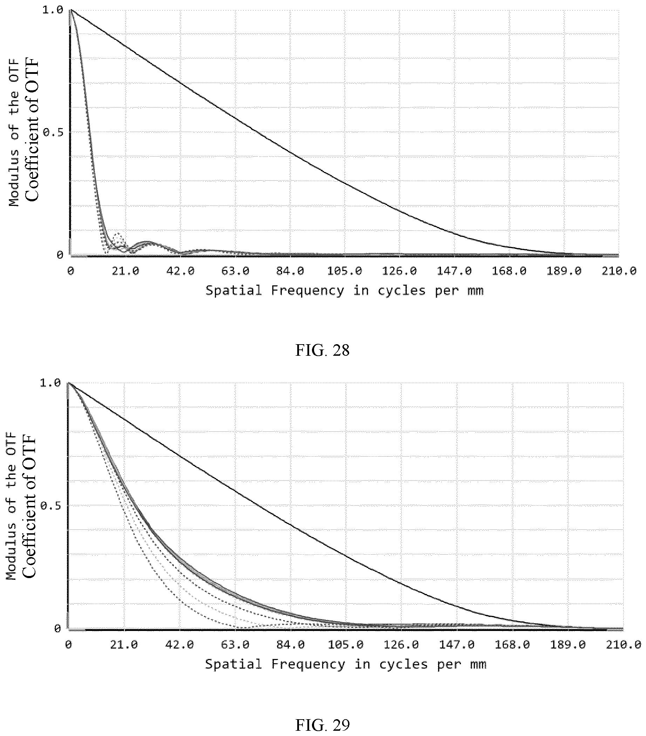

FIGs. 28 to 30 are MTF curves of the combination of the second set of tube lenses 41 with the objective lens 10 when the refractive index of the material of the second substrate 203 is 1.717, 1.911, or 2.003, respectively. The MTF curves can be used to evaluate the resolution indicators of the second set of tube lenses 41. In the figures, the abscissa represents the spatial frequency, and the ordinate represents the value of MTF. The maximal value is 1, and a greater value indicates a higher resolution. As can be seen, when the refractive index of the material of the second substrate 203 is 2.003, the MTF curves of the combination of the second set of tube lenses 41 with the objective lens 10 in different fields of view are all close to the diffraction limit at different spatial frequencies, and the optimal resolution is achieved.

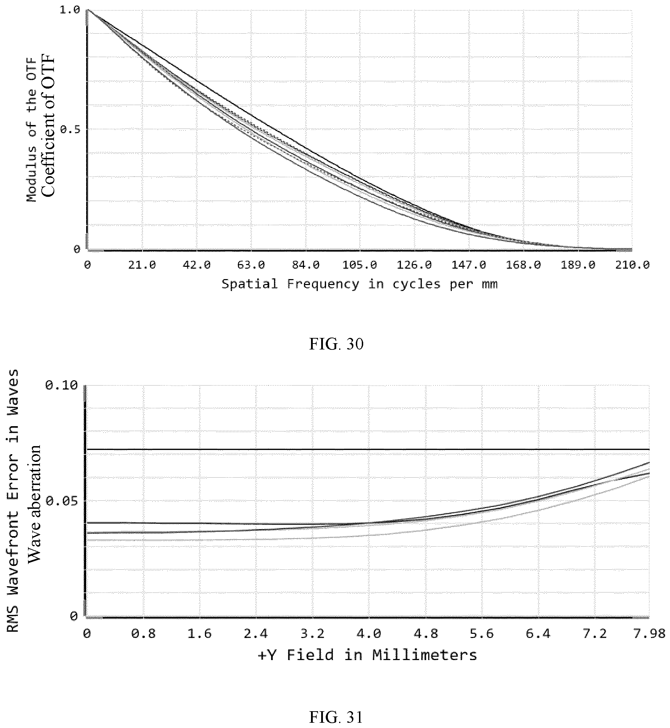

FIG. 31 is a diagram illustrating the wavefront aberration curves of the combination of the second set of tube lenses 41 with the objective lens 10. The wavefront aberration curve is also an indicator for evaluating the image quality of the imaging system 100, and a higher MTF value generally indicates a better wavefront aberration curve. As shown in

FIG. 31, after the second set of tube lenses 41 is combined with the objective lens 10, the wavefront aberrations in different fields of view are lower than the diffraction limit, and the curves are relatively concentrated, achieving a better image quality.

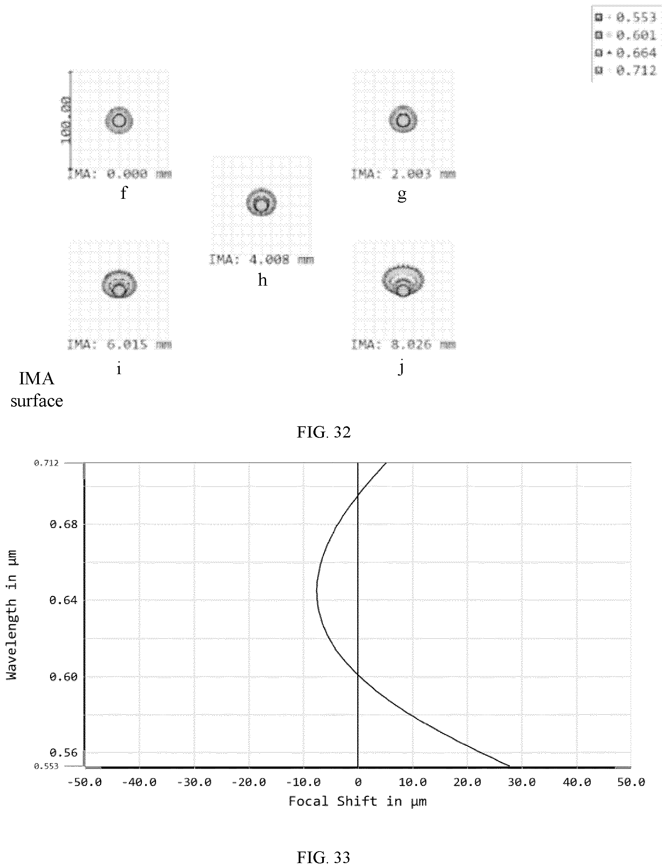

FIG. 32 is a spot diagram of the combination of the second set of tube lenses 41 with the objective lens 10 in 5 different fields of view. The spot diagram is a spot pattern observed at the image side of the spots in the field of view at the object side. The spot diagram has a diffraction-limited circle with an airy radius of 6.392 μm, and the root mean square (RMS) radii of the spots in the fields of view coded f-j are 5.965 μm, 6.121 μm, 6.588 μm, 7.531 μm, and 9.428 μm, respectively. It can be seen that, in the second set of tube lenses 41 according to embodiments of the present application, the spots of the 5 fields of view are all within or close to the diffraction-limited circle. The spot size of the second set of tube lenses 41 reaches the diffraction limit in the fields of view, and a better image quality is achieved, suggesting good aberration of the imaging system 100 comprising the second set of tube lenses 41.

FIG. 33 is a diagram illustrating the focal shift curve of the combination of the second set of tube lenses 41 with the objective lens 10. The focal shift diagram is a positional diagram of the focuses of light beams of different wavelengths. It can be seen from the figure that the maximum focal shift range, or axial chromatic aberration, from 553 nm to 712 nm is 35.3522 μm.

In one example, the imaging system 100 is applied in such a way that: when the light source 60 emits excitation light of a first waveband (with a wavelength of, e.g., 660 nm), the excitation light excites the first surface 210 and the second surface 220 to generate a base A fluorescence signal and a base C fluorescence signal. The base A fluorescence signal and the base C fluorescence signal are acquired by the objective lens 10, transmitted by the second dichroic mirror 61, reflected by the mirror 54, and separated by the first dichroic mirror 51. The base A fluorescence signal of the first surface 210 is reflected by the first dichroic mirror 51, reflected by the first beamsplitter 52, and received by the first image sensor 211, and the base A fluorescence signal of the second surface 220 is reflected by the first dichroic mirror 51, transmitted by the first beamsplitter 52, and received by the third image sensor 221; the base C fluorescence signal of the first surface 210 is transmitted by the first dichroic mirror 51, reflected by the second beamsplitter 53, and received by the second image sensor 212, and the base C fluorescence signal of the second surface 220 is transmitted by the first dichroic mirror 51, transmitted by the second beamsplitter 53, and received by the fourth image sensor 222. When the light source 60 emits excitation light of a second waveband (with a wavelength of, e.g., 532 nm), the excitation light excites the first surface 210 and the second surface 220 to generate a base G fluorescence signal and a base T fluorescence signal. The base G fluorescence signal and the base T fluorescence signal are acquired by the objective lens 10, transmitted by the second dichroic mirror 61, reflected by the mirror 54, and separated by the first dichroic mirror 51. The base G fluorescence signal of the first surface 210 is reflected by the first dichroic mirror 51, reflected by the first beamsplitter 52, and received by the first image sensor 211, and the base G fluorescence signal of the second surface 220 is reflected by the first dichroic mirror 51, transmitted by the first beamsplitter 52, and received by the third image sensor 221; the base T fluorescence signal of the first surface 210 is transmitted by the first dichroic mirror 51, reflected by the second beamsplitter 53, and received by the second image sensor 212, and the base T fluorescence signal of the second surface 220 is transmitted by the first dichroic mirror 51, transmitted by the second beamsplitter 53, and received by the fourth image sensor 222.

The sequencing system according to embodiments of the present application comprises the imaging system 100. The imaging system 100 of the present disclosure can simultaneously achieve the acquisition of optical signals on two surfaces of a sample 200 of interest and imaging. This allows the imaging system 100 to simultaneously sequence bases on the two surfaces of the sample 200 of interest during the gene sequencing process, thus reducing the sequencing time and improving the sequencing efficiency. The sequencing system according to embodiments of the present application includes, but is not limited to, systems having sequencing functionality such as gene sequencing systems and nucleic acid sequencing systems, while other systems manufactured as per the same principle are also applicable to embodiments of the present application.

The invention is further characterized by the following items:

1. An imaging system for simultaneously imaging a first surface and a second surface of a sample of interest, comprising:

   an objective lens;
   image sensors, comprising a first set of image sensors and a second set of image sensors,
   wherein a first set of tube lenses are arranged between the objective lens and the first set of image sensors and are configured for enabling the imaging system to have a first depth of field when imaging the first surface;
   a second set of tube lenses are arranged between the objective lens and the second set of image sensors and are configured for enabling the imaging system to have a second depth of field when imaging the second surface;
   the first depth of field and the second depth of field are both smaller than a distance of displacement from the first surface to the second surface along the optical axis of the objective lens.

2. The imaging system according to item 1, wherein the first depth of field ranges from 1.5 μm to 1.8 μm; and/or,
the second depth of field ranges from 1.5 μm to 1.8 μm.
3. The imaging system according to item 1 or 2, wherein the distance of displacement from the first surface to the second surface along the optical axis of the objective lens ranges from 40 μm to 60 μm.
4. The imaging system according to any one of items 1-3, wherein the objective lens has a numerical aperture greater than 0.6 and a field of view greater than 1.2 mm.
5. The imaging system according to any one of items 1-4, wherein the combination of the objective lens and the first set of tube lenses has a distortion of less than 0.5.

6. The imaging system according to any one of items 1-4, wherein the combination of the objective lens and the second set of tube lenses has a distortion of less than 0.2.

7. The imaging system according to any one of items 1-6, wherein the first set of tube lenses comprise, in sequence:

a first optical lens, having a negative refractive power;
a second optical lens, having a positive refractive power;
a third optical lens, having a positive refractive power;
a fourth optical lens, having a positive refractive power; and
a fifth optical lens, having a negative refractive power;
the object-side surfaces and the image-side surfaces of the first optical lens, the second optical lens, the third optical lens, the fourth optical lens, and the fifth optical lens are all spherical surfaces, and the object-side surface of one optical lens and the image-side surface of the other optical lens in each pair of adjacent optical lenses are arranged towards each other.

8. The imaging system according to item 7, wherein the object-side surface of the first optical lens is convex at the optical axis of the first optical lens, and the image-side surface of the first optical lens is concave at the optical axis of the first optical lens;
the object-side surface of the first optical lens has a curvature radius of 160 mm to 190 mm at the optical axis of the first optical lens; and/or the image-side surface of the first optical lens has a curvature radius of 60 mm to 90 mm at the optical axis of the first optical lens.

9. The imaging system according to item 7 or 8, wherein the object-side surface of the second optical lens is convex at the optical axis of the second optical lens, and the image-side surface of the second optical lens is convex at the optical axis of the second optical lens;
the object-side surface of the second optical lens has a curvature radius of 60 mm to 90 mm at the optical axis of the second optical lens; and/or the image-side surface of the second optical lens has a curvature radius of -170 mm to -140 mm at the optical axis of the second optical lens.

10. The imaging system according to any one of items 7-9, wherein the object-side surface of the third optical lens is convex at the optical axis of the third optical lens, and the image-side surface of the third optical lens is concave at the optical axis of the third optical lens;
the object-side surface of the third optical lens has a curvature radius of 40 mm to 70 mm at the optical axis of the third optical lens; and/or the image-side surface of the third optical lens has a curvature radius of 110 mm to 140 mm at the optical axis of the third optical lens.

11. The imaging system according to any one of items 7-10, wherein the object-side surface of the fourth optical lens is convex at the optical axis of the fourth optical lens, and the image-side surface of the fourth optical lens is convex at the optical axis of the fourth optical lens;
the object-side surface of the fourth optical lens has a curvature radius of 30 mm to 50 mm at the optical axis of the fourth optical lens; and/or the image-side surface of the fourth optical lens has a curvature radius of -700 mm to -650 mm at the optical axis of the fourth optical lens.

12. The imaging system according to any one of items 7-11, wherein the object-side surface of the fifth optical lens is concave at the optical axis of the fifth optical lens, and the image-side surface of the fifth optical lens is concave at the optical axis of the fifth optical lens;
the object-side surface of the fifth optical lens has a curvature radius of -700 mm to -650 mm at the optical axis of the fifth optical lens; and/or the image-side surface of the fifth optical lens has a curvature radius of 20 mm to 40 mm at the optical axis of the fifth optical lens.

13. The imaging system according to any one of items 7-12, wherein the first optical lens has a thickness of 2 mm to 5 mm at the optical axis of the first optical lens; and/or,

the second optical lens has a thickness of 9 mm to 13 mm at the optical axis of the second optical lens; and/or,
the third optical lens has a thickness of 6 mm to 10 mm at the optical axis of the third optical lens; and/or,
the fourth optical lens has a thickness of 9 mm to 14 mm at the optical axis of the fourth optical lens; and/or,
the fifth optical lens has a thickness of 3 mm to 7 mm at the optical axis of the fifth optical lens.

14. The imaging system according to any one of items 7-13, wherein the first optical lens and the second optical lens are cemented to form an optical lens set; and/or, the fourth optical lens and the fifth optical lens are cemented to form an optical lens set.

15. The imaging system according to any one of items 1-14, wherein the second set of tube lenses comprise, in sequence:

a sixth optical lens, having a negative refractive power;

a seventh optical lens, having a positive refractive power;
an eighth optical lens, having a positive refractive power;
a ninth optical lens, having a positive refractive power; and
a tenth optical lens, having a negative refractive power;
the object-side surfaces and the image-side surfaces of the sixth optical lens, the seventh optical lens, the eighth optical lens, the ninth optical lens, and the tenth optical lens are all spherical surfaces, and the object-side surface of one lens and the image-side surface of the other lens in each pair of adjacent lenses are arranged towards each other.

16. The imaging system according to item 15, wherein the object-side surface of the sixth optical lens is convex at the optical axis of the sixth optical lens, and the image-side surface of the sixth optical lens is concave at the optical axis of the sixth optical lens;
the object-side surface of the sixth optical lens has a curvature radius of 110 mm to 130 mm at the optical axis of the sixth optical lens; the image-side surface of the sixth optical lens has a curvature radius of 40 mm to 70 mm at the optical axis of the sixth optical lens.
17. The imaging system according to item 15 or 16, wherein the object-side surface of the seventh optical lens is convex at the optical axis of the seventh optical lens, and the image-side surface of the seventh optical lens is convex at the optical axis of the seventh optical lens;
the object-side surface of the seventh optical lens has a curvature radius of 40 mm to 70 mm at the optical axis of the seventh optical lens; the image-side surface of the seventh optical lens has a curvature radius of -350 mm to -300 mm at the optical axis of the seventh optical lens.
18. The imaging system according to any one of items 15-17, wherein the object-side surface of the eighth optical lens is convex at the optical axis of the eighth optical lens, and the image-side surface of the eighth optical lens is concave at the optical axis of the eighth optical lens;
the object-side surface of the eighth optical lens has a curvature radius of 50 mm to 70 mm at the optical axis of the eighth optical lens; the image-side surface of the eighth optical lens has a curvature radius of 180 mm to 220 mm at the optical axis of the eighth optical lens.
19. The imaging system according to any one of items 15-18, wherein the object-side surface of the ninth optical lens is convex at the optical axis of the ninth optical lens, and the image-side surface of the ninth optical lens is convex at the optical axis of the ninth optical lens;
the object-side surface of the ninth optical lens has a curvature radius of 40 mm to 60 mm at the optical axis of the ninth optical lens; the image-side surface of the ninth optical lens has a curvature radius of -150 mm to -110 mm at the optical axis of the ninth optical lens.
20. The imaging system according to any one of items 15-19, wherein the object-side surface of the tenth optical lens is concave at the optical axis of the tenth optical lens, and the image-side surface of the tenth optical lens is concave at the optical axis of the tenth optical lens;
the object-side surface of the tenth optical lens has a curvature radius of -150 mm to -110 mm at the optical axis of the tenth optical lens; the image-side surface of the tenth optical lens has a curvature radius of 20 mm to 50 mm at the optical axis of the tenth optical lens.
21. The imaging system according to any one of items 15-20, wherein the sixth optical lens has a thickness of 3 mm to 7 mm at the optical axis of the sixth optical lens;

the seventh optical lens has a thickness of 9 mm to 13 mm at the optical axis of the seventh optical lens;
the eighth optical lens has a thickness of 7 mm to 10 mm at the optical axis of the eighth optical lens;
the ninth optical lens has a thickness of 10 mm to 15 mm at the optical axis of the ninth optical lens;
the tenth optical lens has a thickness of 4 mm to 7 mm at the optical axis of the tenth optical lens.

22. The imaging system according to any one of items 16-20, wherein the sixth optical lens and the seventh optical lens are cemented to form an optical lens set, and the ninth optical lens and the tenth optical lens are cemented to form an optical lens set.
23. The imaging system according to any one of items 1-22, wherein

a beamsplitter assembly is arranged between the objective lens and the image sensors;
the beamsplitter assembly is configured for splitting optical signals acquired by the objective lens, so as to simultaneously deliver a first optical signal generated by the first surface to the first set of image sensors and a second optical signal generated by the second surface to the second set of image sensors.

24. The imaging system according to item 23, wherein

the first set of image sensors at least comprise a first image sensor and a second image sensor;
the first set of tube lenses at least comprise a first tube lens and a second tube lens that are identical;

the beamsplitter assembly is configured for splitting the first optical signal into a light beam with a first wavelength and a light beam with a second wavelength, and simultaneously delivering the light beam with the first wavelength to the first image sensor through the first tube lens and the light beam with the second wavelength to the second image sensor through the second tube lens.

25. The imaging system according to item 23 or 24, whereinthe second set of image sensors at least comprise a third image sensor and a fourth image sensor;

the second set of tube lenses at least comprise a third tube lens and a fourth tube lens that are identical;

the beamsplitter assembly is configured for splitting the second optical signal into a light beam with a third wavelength and a light beam with a fourth wavelength, and simultaneously delivering the light beam with the third wavelength to the third image sensor through the third tube lens and the light beam with the fourth wavelength to the fourth image sensor through the fourth tube lens.

26. The imaging system according to item 24 or 25, wherein

the beamsplitter assembly comprises a first dichroic mirror, a first beamsplitter, and a second beamsplitter;

the first dichroic mirror is configured for reflecting the light beam with the first wavelength to the first beamsplitter and transmitting the light beam with the second wavelength to the second beamsplitter;

the first beamsplitter is configured for receiving the light beam with the first wavelength reflected from the first dichroic mirror and reflecting the light beam with the first wavelength to the first tube lens, so as to deliver the light beam with the first wavelength to the first image sensor through the first tube lens;

the second beamsplitter is configured for receiving the light beam with the second wavelength transmitted from the first dichroic mirror and reflecting the light beam with the second wavelength to the second tube lens, so as to deliver the light beam with the second wavelength to the second image sensor through the second tube lens.

27. The imaging system according to item 26, wherein

the first dichroic mirror is configured for reflecting the light beam with the third wavelength to the first beamsplitter and transmitting the light beam with the fourth wavelength to the second beamsplitter;

the first beamsplitter is configured for receiving the light beam with the third wavelength reflected from the first dichroic mirror and transmitting the light beam with the third wavelength to the third tube lens, so as to deliver the light beam with the third wavelength to the third image sensor through the third tube lens;

the second beamsplitter is configured for receiving the light beam with the fourth wavelength transmitted from the first dichroic mirror and transmitting the light beam with the fourth wavelength to the fourth tube lens, so as to deliver the light beam with the fourth wavelength to the fourth image sensor through the fourth tube lens.

28. The imaging system according to any one of items 1-27, comprising a light source and a second dichroic mirror arranged between the light source and the objective lens, wherein the second dichroic mirror is arranged on the optical axis of the objective lens, the light source is configured for emitting an excitation light beam, and the second dichroic mirror is configured for reflecting the excitation light beam toward the objective lens, such that the excitation light beam is projected through the objective lens to the first surface and the second surface.

29. The imaging system according to any one of items 1 to 28, comprising an autofocus apparatus, wherein the autofocus apparatus is configured for detecting the positional relationship between the sample of interest and the objective lens and for positioning the sample of interest in the focal plane of the objective lens according to the detected relative positions of the sample of interest and the objective lens.

30. A sequencing system, comprising the imaging system according to any one of items 1-29.

In the description of this specification, the description of the terms "one embodiment", "some embodiments", "schematic embodiments", "examples", "specific examples", "some examples", or the like, means that the particular features, structures, materials, or characteristics described in the embodiments or examples are included in at least one embodiment or example of the present disclosure. In this specification, the schematic description of the aforementioned terms does not necessarily refer to the same embodiment or example. Moreover, the particular features, structures, materials, or characteristics described may be combined in any embodiment or example in any appropriate manner.

Although the embodiments of the present application have been illustrated and described, it will be appreciated by those of ordinary skills in the art that various changes, modifications, replacements, and variations can be made to these embodiments without departing from the principle and purpose of the present application, and the scope of the present application is defined by the claims and equivalents therefore.

## Claims

**1.** An imaging system for simultaneously imaging a first surface and a second surface of a sample of interest, comprising:

an objective lens;
image sensors, comprising a first set of image sensors and a second set of image sensors, wherein a first set of tube lenses are arranged between the objective lens and the first set of image sensors and are configured for enabling the imaging system to have a first depth of field when imaging the first surface;
a second set of tube lenses are arranged between the objective lens and the second set of image sensors and are configured for enabling the imaging system to have a second depth of field when imaging the second surface;
the first depth of field and the second depth of field are both smaller than a distance of displacement from the first surface to the second surface along the optical axis of the objective lens.

**2.** The imaging system according to claim 1, wherein the first depth of field ranges from 1.5 μm to 1.8 μm; and/or, the second depth of field ranges from 1.5 μm to 1.8 μm.

**3.** The imaging system according to claim 1 or 2, wherein the distance of displacement from the first surface to the second surface along the optical axis of the objective lens ranges from 40 μm to 60 μm.

**4.** The imaging system according to any one of claims 1-3, wherein the objective lens has a numerical aperture greater than 0.6 and a field of view greater than 1.2 mm.

**5.** The imaging system according to any one of claims 1-4, wherein the combination of the objective lens and the first set of tube lenses has a distortion of less than 0.5.

**6.** The imaging system according to any one of claims 1-4, wherein the combination of the objective lens and the second set of tube lenses has a distortion of less than 0.2.

**7.** The imaging system according to any one of claims 1-6, wherein the first set of tube lenses comprise, in sequence:

a first optical lens, having a negative refractive power;
a second optical lens, having a positive refractive power;
a third optical lens, having a positive refractive power;
a fourth optical lens, having a positive refractive power; and
a fifth optical lens, having a negative refractive power;
the object-side surfaces and the image-side surfaces of the first optical lens, the second optical lens, the third optical lens, the fourth optical lens, and the fifth optical lens are all spherical surfaces, and the object-side surface of one optical lens and the image-side surface of the other optical lens in each pair of adjacent optical lenses are arranged towards each other.

**8.** The imaging system according to claim 7, wherein the object-side surface of the first optical lens is convex at the optical axis of the first optical lens, and the image-side surface of the first optical lens is concave at the optical axis of the first optical lens;

the object-side surface of the first optical lens has a curvature radius of 160 mm to 190 mm at the optical axis of the first optical lens; and/or the image-side surface of the first optical lens has a curvature radius of 60 mm to 90 mm at the optical axis of the first optical lens;
the object-side surface of the second optical lens is convex at the optical axis of the second optical lens, and the image-side surface of the second optical lens is convex at the optical axis of the second optical lens;
the object-side surface of the second optical lens has a curvature radius of 60 mm to 90 mm at the optical axis of the second optical lens; and/or the image-side surface of the second optical lens has a curvature radius of -170 mm to -140 mm at the optical axis of the second optical lens;
the object-side surface of the third optical lens is convex at the optical axis of the third optical lens, and the image-side surface of the third optical lens is concave at the optical axis of the third optical lens;
the object-side surface of the third optical lens has a curvature radius of 40 mm to 70 mm at the optical axis of the third optical lens; and/or the image-side surface of the third optical lens has a curvature radius of 110 mm to 140 mm at the optical axis of the third optical lens;
the object-side surface of the fourth optical lens is convex at the optical axis of the fourth optical lens, and the image-side surface of the fourth optical lens is convex at the optical axis of the fourth optical lens;
the object-side surface of the fourth optical lens has a curvature radius of 30 mm to 50 mm at the optical axis of the fourth optical lens; and/or the image-side surface of the fourth optical lens has a curvature radius of -700 mm to -650 mm at the optical axis of the fourth optical lens;
the object-side surface of the fifth optical lens is concave at the optical axis of the fifth optical lens, and the image-side surface of the fifth optical lens is concave at the optical axis of the fifth optical lens;
the object-side surface of the fifth optical lens has a curvature radius of -700 mm to -650 mm at the optical axis of the fifth optical lens; and/or the image-side surface of the fifth optical lens has a curvature radius of 20 mm to 40 mm at the optical axis of the fifth optical lens;
optionally, the first optical lens has a thickness of 2 mm to 5 mm at the optical axis of the first optical lens; and/or,
the second optical lens has a thickness of 9 mm to 13 mm at the optical axis of the second optical lens; and/or,
the third optical lens has a thickness of 6 mm to 10 mm at the optical axis of the third optical lens; and/or,
the fourth optical lens has a thickness of 9 mm to 14 mm at the optical axis of the fourth optical lens; and/or,
the fifth optical lens has a thickness of 3 mm to 7 mm at the optical axis of the fifth optical lens;
optionally, the first optical lens and the second optical lens are cemented to form an optical lens set; and/or, the fourth optical lens and the fifth optical lens are cemented to form an optical lens set.

**9.** The imaging system according to any one of claims 1-8, wherein the second set of tube lenses comprise, in sequence:

a sixth optical lens, having a negative refractive power;
a seventh optical lens, having a positive refractive power;
an eighth optical lens, having a positive refractive power;
a ninth optical lens, having a positive refractive power; and
a tenth optical lens, having a negative refractive power;
the object-side surfaces and the image-side surfaces of the sixth optical lens, the seventh optical lens, the eighth optical lens, the ninth optical lens, and the tenth optical lens are all spherical surfaces, and the object-side surface of one lens and the image-side surface of the other lens in each pair of adjacent lenses are arranged towards each other。

**10.** The imaging system according to claim 9, wherein the object-side surface of the sixth optical lens is convex at the optical axis of the sixth optical lens, and the image-side surface of the sixth optical lens is concave at the optical axis of the sixth optical lens;

the object-side surface of the sixth optical lens has a curvature radius of 110 mm to 130 mm at the optical axis of the sixth optical lens; the image-side surface of the sixth optical lens has a curvature radius of 40 mm to 70 mm at the optical axis of the sixth optical lens;
the object-side surface of the seventh optical lens is convex at the optical axis of the seventh optical lens, and the image-side surface of the seventh optical lens is convex at the optical axis of the seventh optical lens;
the object-side surface of the seventh optical lens has a curvature radius of 40 mm to 70 mm at the optical axis of the seventh optical lens; the image-side surface of the seventh optical lens has a curvature radius of -350 mm to -300 mm at the optical axis of the seventh optical lens;
the object-side surface of the eighth optical lens is convex at the optical axis of the eighth optical lens, and the image-side surface of the eighth optical lens is concave at the optical axis of the eighth optical lens;
the object-side surface of the eighth optical lens has a curvature radius of 50 mm to 70 mm at the optical axis of the eighth optical lens; the image-side surface of the eighth optical lens has a curvature radius of 180 mm to 220 mm at the optical axis of the eighth optical lens;
the object-side surface of the ninth optical lens is convex at the optical axis of the ninth optical lens, and the image-side surface of the ninth optical lens is convex at the optical axis of the ninth optical lens;
the object-side surface of the ninth optical lens has a curvature radius of 40 mm to 60 mm at the optical axis of the ninth optical lens; the image-side surface of the ninth optical lens has a curvature radius of -150 mm to -110 mm at the optical axis of the ninth optical lens;
the object-side surface of the tenth optical lens is concave at the optical axis of the tenth optical lens, and the image-side surface of the tenth optical lens is concave at the optical axis of the tenth optical lens;

the object-side surface of the tenth optical lens has a curvature radius of -150 mm to -110 mm at the optical axis of the tenth optical lens; the image-side surface of the tenth optical lens has a curvature radius of 20 mm to 50 mm at the optical axis of the tenth optical lens;

optionally, the sixth optical lens has a thickness of 3 mm to 7 mm at the optical axis of the sixth optical lens;

the seventh optical lens has a thickness of 9 mm to 13 mm at the optical axis of the seventh optical lens;

the eighth optical lens has a thickness of 7 mm to 10 mm at the optical axis of the eighth optical lens;

the ninth optical lens has a thickness of 10 mm to 15 mm at the optical axis of the ninth optical lens;

the tenth optical lens has a thickness of 4 mm to 7 mm at the optical axis of the tenth optical lens;

optionally, the sixth optical lens and the seventh optical lens are cemented to form an optical lens set, and the ninth optical lens and the tenth optical lens are cemented to form an optical lens set.

**11.** The imaging system according to any one of claims 1-10, wherein a beamsplitter assembly is arranged between the objective lens and the image sensors; the beamsplitter assembly is configured for splitting optical signals acquired by the objective lens, so as to simultaneously deliver a first optical signal generated by the first surface to the first set of image sensors and a second optical signal generated by the second surface to the second set of image sensors.

**12.** The imaging system according to claim 11, wherein the first set of image sensors at least comprise a first image sensor and a second image sensor;

the first set of tube lenses at least comprise a first tube lens and a second tube lens that are identical;

the beamsplitter assembly is configured for splitting the first optical signal into a light beam with a first wavelength and a light beam with a second wavelength, and simultaneously delivering the light beam with the first wavelength to the first image sensor through the first tube lens and the light beam with the second wavelength to the second image sensor through the second tube lens.

**13.** The imaging system according to claim 12, wherein the second set of image sensors at least comprise a third image sensor and a fourth image sensor;

the second set of tube lenses at least comprise a third tube lens and a fourth tube lens that are identical;

the beamsplitter assembly is configured for splitting the second optical signal into a light beam with a third wavelength and a light beam with a fourth wavelength, and simultaneously delivering the light beam with the third wavelength to the third image sensor through the third tube lens and the light beam with the fourth wavelength to the fourth image sensor through the fourth tube lens.

**14.** The imaging system according to claim 13, wherein the beamsplitter assembly comprises a first dichroic mirror, a first beamsplitter, and a second beamsplitter;

the first dichroic mirror is configured for reflecting the light beam with the first wavelength to the first beamsplitter and transmitting the light beam with the second wavelength to the second beamsplitter;

the first beamsplitter is configured for receiving the light beam with the first wavelength reflected from the first dichroic mirror and reflecting the light beam with the first wavelength to the first tube lens, so as to deliver the light beam with the first wavelength to the first image sensor through the first tube lens;

the second beamsplitter is configured for receiving the light beam with the second wavelength transmitted from the first dichroic mirror and reflecting the light beam with the second wavelength to the second tube lens, so as to deliver the light beam with the second wavelength to the second image sensor through the second tube lens;

the first dichroic mirror is also configured for reflecting the light beam with the third wavelength to the first beamsplitter and transmitting the light beam with the fourth wavelength to the second beamsplitter;

the first beamsplitter is configured for receiving the light beam with the third wavelength reflected from the first dichroic mirror and transmitting the light beam with the third wavelength to the third tube lens, so as to deliver the light beam with the third wavelength to the third image sensor through the third tube lens;

the second beamsplitter is configured for receiving the light beam with the fourth wavelength transmitted from the first dichroic mirror and transmitting the light beam with the fourth wavelength to the fourth tube lens, so as to deliver the light beam with the fourth wavelength to the fourth image sensor through the fourth tube lens;

optionally, the imaging system comprises a light source and a second dichroic mirror arranged between the light source and the objective lens, wherein the second dichroic mirror is arranged on the optical axis of the objective lens, the light source is configured for emitting an excitation light beam, and the second dichroic mirror is configured for reflecting the excitation light beam toward the objective lens, such that the excitation light beam is projected through the objective lens to the first surface and the second surface;

optionally, the imaging system comprises an autofocus apparatus, wherein the autofocus apparatus is configured for detecting the positional relationship between the sample of interest and the objective lens and for positioning the sample of interest in the focal plane of the objective lens according to the detected relative positions of the sample of interest and the objective lens.

**15.** A sequencing system, comprising the imaging system according to any one of claims 1-14.

100
70
212(21)
302
407(40)
50
62
62
417(41)
51
222(22)
54
53
312
20
211(21)
61
52
301
62
406(40)
62
416(41)
111
60
311
221(22)
10
200

FIG. 1

200
30
31
201
202
210
First depth of field
203
220
204
Second depth of field

FIG. 2

200
201
210
202
220
203

FIG. 3

200
202
210
201
220
204
203

FIG. 4

Wavefront aberration
0.7
0.6
0.5
0.4
0.3
0.2
0.1
0
-1
-0.8
-0.6
-0.4
-0.2
0
0.2
0.4
0.6
0.8
1
Focus
Field 1
Field 2
Field 3
Field 4
Field 5
Field 6
Field 7
Field 8
Field 9
Field 10

FIG. 5

8.0
5.0
0
+ Y field (millimeter)
-0.5
0
0.5
Percent

FIG. 6

8.0
5.0
0
+ Y field (millimeter)
-0.20
0
0.20
Percent

FIG. 7

40
Light propagation direction
401
402
403
404
405
4022
4012
4011
4021
4031
4032
4042
4052
4051
4041
1.0
0.5
0
Modulus of the OTF
Coefficient of OTF
0
22.0
44.0
66.0
88.0
110.0
132.0
154.0
176.0
198.0
220.0
Spatial Frequency in cycles per mm

FIG. 8

FIG. 9

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 10

Freq = 176.5, MTF = 0.6523
1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 11

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 12

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 13

Field = 0.2903, RMS = 0.08571
RMS Wavefront Error in Waves
Wave aberration
0.10
0.05
0
0
0.8
1.6
2.4
3.2
4.0
4.8
5.6
6.4
7.2
8.0
+Y Field in Millimeters

FIG. 14

0.553
0.601
0.664
0.712
20.00
IMA: 0.000 mm
a
IMA: 4.006 mm
b
IMA: 5.671 mm
c
IMA: 6.953 mm
d
IMA: 8.039 mm
e
IMA surface

FIG. 15

0.712
0.68
0.64
0.60
0.56
0.553
Wavelength in µm
-20.0
-16.0
-12.0
-8.0
-4.0
0
4.0
8.0
12.0
16.0
20.0
Focal Shift in µm

FIG. 16

Light propagation direction
41
411
412
413
414
415
4122
4112
4111
4121
4131
4132
4142
4152
4151
4141

FIG. 17

1.0
0.5
0
Modulus of the OTF
Coefficient of OTF
0
22.0
44.0
66.0
88.0
110.0
132.0
154.0
176.0
198.0
220.0
Spatial Frequency in cycles per mm

FIG. 18

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 19

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 20

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 21

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 22

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 23

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 24

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 25

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 26

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 27

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 28

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 29

1.0
0.5
0
Modulus of the OTF
Coefficient of OTF
0
21.0
42.0
63.0
84.0
105.0
126.0
147.0
168.0
189.0
210.0
Spatial Frequency in cycles per mm

FIG. 30

0.10
0.05
0
RMS Wavefront Error in Waves
Wave aberration
0
0.8
1.6
2.4
3.2
4.0
4.8
5.6
6.4
7.2
7.98
+Y Field in Millimeters

FIG. 31

0.553
0.601
0.664
0.712

100.00
IMA: 0.000 mm
f
IMA: 2.003 mm
g
IMA: 4.008 mm
h
IMA: 6.015 mm
i
IMA: 8.026 mm
j
IMA
surface

FIG. 32

0.712
0.68
0.64
0.60
0.56
0.553
Wavelength in µm
-50.0
-40.0
-30.0
-20.0
-10.0
0
10.0
20.0
30.0
40.0
50.0
Focal Shift in µm

FIG. 33

Europäisches
Patentamt
European
Patent Office
Office européen
des brevets

# EUROPEAN SEARCH REPORT

Application Number
EP 24 19 3689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG XIN ET AL: "Double-layer focal plane microscopy for high throughput DNA sequencing", OPTICS EXPRESS, vol. 30, no. 11, 12 May 2022 (2022-05-12), page 18496, XP093229398, US ISSN: 1094-4087, DOI: 10.1364/OE.454100 | 1-7,9, 11-15 | INV. G02B21/18 G02B21/36 G02B27/00 G02B27/10 |
| Y | * abstract; p. 18498, second paragraph; | 7,9, 11-14 | |
| A | figures 2, 5 * | 8,10 | |
| | ----- | | |
| X | WO 2023/004014 A1 (ELEMENT BIOSCIENCES INC [US]; GHORBANI ARASH [US] ET AL.) 26 January 2023 (2023-01-26) | 1-7,9, 11-15 | |
| Y | * paragraphs [0247] - [0251], [0259] - [0263], [0319], [0324], [0632]; figures 3A, 3B * | 7,9, 11-14 | |
| | ----- | | |
| Y | US 5 701 196 A (NAKAMURA SHINICHI [JP]) 23 December 1997 (1997-12-23) * abstract; claim 1; figures 10A, 10B; example 10 * | 7,9 | TECHNICAL FIELDS SEARCHED (IPC) |
| | ----- | | G02B |
| Y | US 2005/117214 A1 (WARTMANN ROLF [DE] ET AL) 2 June 2005 (2005-06-02) * paragraph [0101]; figure 1 * | 7,9 | |
| | ----- | | |
| A | ENGELHARDT MORITZ ET AL: "Mapping volumes to planes: Camera-based strategies for snapshot volumetric microscopy", FRONTIERS IN PHYSICS, vol. 10, 11 October 2022 (2022-10-11), XP093126030, ISSN: 2296-424X, DOI: 10.3389/fphy.2022.1010053 * section "2 Multiplane imaging" * | 11-14 | |
| | ----- | | |
| | -/-- | | |

The present search report has been drawn up for all claims

1

| Place of search | Date of completion of the search | Examiner |
|---|---|---|
| Munich | 2 December 2024 | Kaiser, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 24 19 3689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2010/328780 A1 (TOCCI MICHAEL D [US]) 30 December 2010 (2010-12-30) * paragraph [0101]; figure 17 * ----- | 11-14 | |

TECHNICAL FIELDS SEARCHED (IPC)

The present search report has been drawn up for all claims

1

| Place of search | Date of completion of the search | Examiner |
|---|---|---|
| Munich | 2 December 2024 | Kaiser, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 19 3689

This annex lists the patent family members relating to the patent documents cited in the above-mentioned European search report. The members are as contained in the European Patent Office EDP file on
The European Patent Office is in no way liable for these particulars which are merely given for the purpose of information.

02-12-2024

| Patent document cited in search report | Publication date | Patent family member(s) | Publication date |
|---|---|---|---|
| WO 2023004014 A1 | 26-01-2023 | AU 2022316142 A1 | 22-02-2024 |
| | | CA 3226482 A1 | 26-01-2023 |
| | | EP 4373968 A1 | 29-05-2024 |
| | | GB 2626865 A | 07-08-2024 |
| | | GB 2629259 A | 23-10-2024 |
| | | IL 310281 A | 01-03-2024 |
| | | JP 2024530883 A | 27-08-2024 |
| | | KR 20240063107 A | 10-05-2024 |
| | | US 2024200133 A1 | 20-06-2024 |
| | | US 2024201088 A1 | 20-06-2024 |
| | | WO 2023004014 A1 | 26-01-2023 |
| US 5701196 A | 23-12-1997 | NONE | |
| US 2005117214 A1 | 02-06-2005 | AT E326713 T1 | 15-06-2006 |
| | | DE 10344943 A1 | 21-04-2005 |
| | | EP 1519210 A1 | 30-03-2005 |
| | | JP 2005107523 A | 21-04-2005 |
| | | US 2005117214 A1 | 02-06-2005 |
| US 2010328780 A1 | 30-12-2010 | EP 2476021 A2 | 18-07-2012 |
| | | JP 2013504940 A | 07-02-2013 |
| | | US 2010328780 A1 | 30-12-2010 |
| | | WO 2011032028 A2 | 17-03-2011 |

EPO FORM P0459

For more details about this annex : see Official Journal of the European Patent Office, No. 12/82